# EUROPEAN PATENT APPLICATION

(11) **EP 4 461 317 A1**
(43) Date of publication of application: **13.11.2024**
(21) Application number: 23173142.3
(22) Date of filing: 12.05.2023
(51) Int. Cl.: A61K 47/68, A61P 35/00, C07K 16/28

(54) **ANTIBODY-DRUG CONJUGATES COMPRISING AN ANTI-NECTIN-4 ANTIBODY AND BELOTECAN OR A DERIVATIVE THEREOF**

(71) Applicant: Emergence Therapeutics GmbH, 47059 Duisburg (DE)
(72) Inventor: The designation of the inventor has not yet been filed
(74) Representative: Smith, Andrew George

(57) **Abstract**

The present invention relates to novel antibody-drug conjugate comprising a monoclonal antibody or an antigen-binding fragment thereof wherein the monoclonal antibody or the antigen-binding fragment thereof binds to Nectin-4 and wherein the drug is a Belotecan or a derivative thereof.

## Description

The present invention relates to novel antibody-drug conjugates comprising a monoclonal antibody or an antigen-binding fragment thereof wherein the monoclonal antibody or the antigen-binding fragment thereof binds to Nectin-4 and wherein the drug is Belotecan or a derivative thereof.

### BACKGROUND OF THE INVENTION

Nectins are adhesion molecules that help organize epithelial and endothelial junctions and serve as receptors for the entry of the herpes simplex virus, measles virus and poliovirus. They belong to the immunoglobulin superfamily and are homologs of the poliovirus receptor (PVR / CD155), and for this, have also been called poliovirus receptor bound proteins (PRR). To date, five members have been described PVR / CD155, Nectin-1 / PRR1 / CD111, Nectin-2 / PRR2 / CD112, Nectin-3 / PRR3 and Nectin-4 / PRR4. Their ectodomain is made up of three immunoglobulin-like (Ig) -type V, C, C domains that share between 30 and 55% identity in their amino acid sequences.

Expression of Nectin / PRR molecules is generally extensive in tissues, including hematopoietic, neuronal, endothelial and epithelial cells, with the exception of Nectin-3 and -4, which exhibit more restricted expression profiles.

Nectin-4 is a particularly interesting target. It is expressed during fetal development, but its expression decreases and is very restricted in adult tissues in comparison to that of other members of the Nectin family. Nectin-4 is a tumor associated antigen in 83 % of bladder cancers, 78 % of breast cancers (mainly triple negative and ERBB2+), 71 % of pancreatic cancers, 55 % of lung cancers, 57% of ovarian cancers, 59% of head and neck cancers, and 55% of esophageal cancers.

Expression of Nectin-4 in these pathologies is associated with poor prognosis, probably as a consequence of the ability of Nectin-4 to confer to tumor cells *in vitro,* higher capacities of migration, proliferation and formation of metastases. In normal tissues, Nectin-4 is only detected in skin, salivary glands, urinary bladder and esophagus. The recent approval by heath authorities of Enfortumab vedotin for the 2^{nd} line treatment of advanced urothelial cancer has completed the validation of Nectin-4 as a target for the treatment of cancer.

### SUMMARY OF THE INVENTION

A first aspect of the present invention is an antibody-drug conjugate of Formula (I)

D-M-V (I),

or pharmaceutically acceptable salt thereof,
wherein
D is Belotecan or a derivative thereof represented by formula (II) 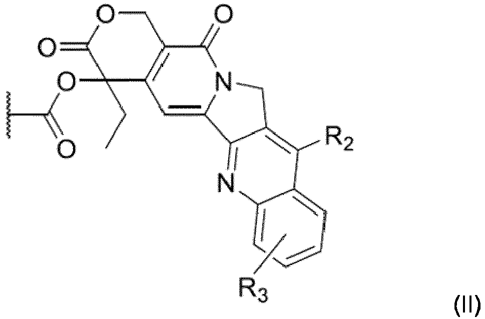 wherein
   - R²: is -(CH₂)₂NR¹CH(CH₃)₂
   - R¹: is H or -SO₂CH₃
   - R³: is H, amino, or C₁-C₆ alkyl, preferably H

   - M: is a linker conjugating D to V, and
   - V: is an anti-Nectin-4 antibody or antigen-binding fragment thereof.

The waved line in formula (II) denotes the position of attachment to the linker M.

Preferred embodiments of D, M and/or V are described herein below.

According to one preferred embodiment the conjugating linker M is a cleavable linker, i.e., a linker cleavable under physiological conditions, e.g., by physiological enzymes. Specific examples of cleavable linkers are peptide-based linkers, which may be subject to cleavage by a protease, or glycoside-based linkers, which may be subject to cleavage by a glycosidase. Another specific example are branched tandem-cleavage linkers that combine moieties that can be cleaved under different conditions or by different enzymes, e.g. protease-cleavable dipeptide and glycosidase-cleavable sugar moieties. In this way, several identical or different payloads can be connected to the antibody.

In certain embodiments, the linker is a hydrophilic polysarcosine linker e.g., as described by Conilh et at. "Exatecan antibody drug conjugates based on a hydrophilic polysarcosine drug-linker platform" (Pharmaceuticals 14 (2021), 247). Further preferred linkers include linkers comprising at least one ethylene glycol unit, e.g., 1, 2, 3, 4, 5, 6, 7, 8, 9, or 10 or more ethylene glycol units, e.g., the linker of the antibody-drug conjugate MEDI7247, an mcc-triazole spacer-PEG7-x-Lys-PABC glycol linker from Trodelvy^{®}, Further preferred linkers include oligopeptide, particularly di- to decapeptide, e.g., tetrapeptide sequences such as glycine-glycine-phenylalanine-glycine from Enhertu^{®}. Further preferred linkers include highly polar spacers such as an acyl group, carbamoyl group and/or sulfamide group added to at least at least one ethylene glycol unit, e.g., 1, 2, 3, 4, 5, 6, 7, 8, 9, or 10 or more ethylene glycol units, e.g., the linker technology called Hydraspace^{™}.

According to another preferred embodiment, the conjugating linker M is of formula (III)

L-A-E (III),

i.e. resulting in ADCs characterized by Formula (IV)

   D-L-A-E-V (IV),
wherein
   - L: is
   -(C₁-C₆-alkylene)-(O-CH₂)ₘ-NR¹-,
   -(C₁-C₆-alkylene)-NR¹-, or
   -(C₁-C₅-alkylene)-NR²C(C=O)-(C₁-C₅-alyklene)-(O-CH₂)ₘ-NR¹-,
   wherein
      R¹ and R² are each independently -H, C₁-C₃-alkyl, in particular methyl, or benzyl,
      m is an integer from 1 to 6, in particular 1,
   - A: is a peptide comprising 2 to 8 amino acids, preferably 2 to 4 amino acids, wherein A is substituted with at least one polyol, wherein polyol is -(C₁-C₆ alkylene)-X^{a}-Y^{b},
   wherein: X^{a}
   is -NR^{c}C(=O)- or -C(=O)NR^{c}-;
   Y^{b} is -CH₂-(C*₁-C*₅-alkyl)-, each C* substituted with a OH group,
   R^{c} is -H, C₁-C₆-alkyl, C₁-C₆ fluoroalkyl, C₃-C₆ cycloalkyl, aryl, heteroaryl, or benzyl,
   - E: is -C(=O)-(C₁-C₁₀-alkylene)-Q-, wherein Q is an antibody coupling group and
   - V and D: are as described herein.

A particularly preferred ADC of the invention is illustrated by formula (V): 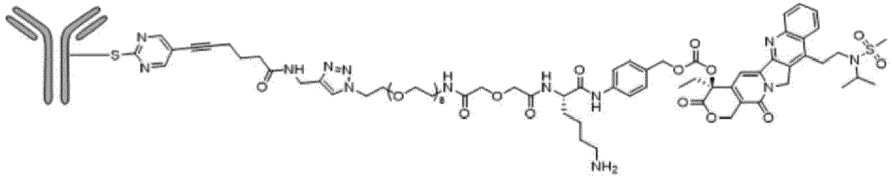 wherein 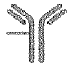 represents the anti-Nectin-4 antibody or antigen-binding fragment thereof (i.e. V).

In another specific embodiment, binding of the conjugating linker M to the antibody can take place, for example, via aldehyde groups that may have been previously introduced into the antibody. For this purpose, the ligand may include a Hydrazine-iso-Picted-Spengler (HIPS) chemistry, the binding of which to the aldehyde group leads to the formation of a stable C-C bond. This HIPS chemistry can be combined with any of the linker types described above.

A further aspect of the present invention is a pharmaceutical composition comprising an active agent, which is an ADC as herein described, wherein the monoclonal antibody or the antigen-binding fragment thereof binds to Nectin-4 and wherein the drug is Belotecan or a derivative thereof, and a pharmaceutically acceptable carrier and/or excipient.

A further aspect of the present invention is the use of an antibody-drug conjugate or a pharmaceutical composition as described above in medicine, particularly in human medicine.

A further aspect of the present invention is the use of an antibody-drug conjugate or a pharmaceutical composition as described above in a method for the prevention and/or treatment of a Nectin-4 associated disorder, particularly for the prevention and/or treatment of a Nectin-4 positive cancer and/or Nectin-4 associated inflammation disorder.

A further aspect of the present invention is the use of an antibody-drug conjugate or a pharmaceutical composition as described above in a method for the prevention and/or treatment of cancer, particularly for the prevention and/or treatment of a cancer selected from bladder, urothelial, endometrial, cervical, colorectal, liver, thyroid, breast, pancreatic, lung, ovarian head and neck and/or esophagus cancer.

A further aspect of the present invention is a method for the prevention and/or treatment of a Nectin-4 associated disorder, particularly for the prevention and/or treatment of a Nectin-4 positive cancer comprising administering to a subject in need thereof a therapeutically effective amount of an antibody-drug conjugate or a pharmaceutical composition as described above.

A further aspect of the present invention is a method for the prevention and/or treatment of cancer, particularly for the prevention and/or treatment of a cancer selected from bladder, urothelial, endometrial, cervical, colorectal, liver, thyroid, breast, pancreatic, lung, ovarian head and neck and/or esophagus cancer comprising administering to a subject in need thereof a therapeutically effective amount of an antibody-drug conjugate or a pharmaceutical composition as described above.

### DETAILED DESCRIPTION OF THE INVENTION

The present inventors have found that conjugates of anti-Nectin-4 antibodies with Belotecan or derivatives thereof demonstrate improved anti-cancer activity compared to the marketed antibody Enfortumab vedotin, which is a conjugate of an anti-Nectin-4 antibody and monomethyl auristatin E (MMAE). Accordingly, the anti-Nectin-4 antibody conjugates of the invention allow an improved treatment of Nectin-4 associated disorders such as Nectin-4 positive cancer.

In particular embodiments, the antibody-drug conjugate of the present invention comprises an anti-Nectin-4 antibody, which specifically binds to Nectin-4 expressed by tumors with a higher affinity in comparison to Nectin-4 expressed by human keratinocytes, e.g., in vitro differentiated Nectin-4 expressing keratinocytes. Preferred examples are the monoclonal antibodies (mAb) 15A7.5, 5A12.2, 9A2.7, 3A1.4 and/or 8F06.

A particularly preferred example is the monoclonal antibody (mAb) 15A7.5 or an antibody derived therefrom. *In vitro,* this selectivity provides mAb 15A7.5 with lower binding affinity, internalization and/or cytotoxic activity towards keratinocytes in comparison to tumor cells and in reference to the activity of the HA22 mAb (Enfortumab), in the same assays.

In particular, the conjugate of the present invention comprises a humanized antibody that derives from the monoclonal anti-Nectin-4 antibody 15A7.5 mAb.

*In vivo,* this low binding capacity to keratinocytes endows mAb 15A7.5 with a higher half-life due to a lower absorption rate in the skin.

In a xenograft mouse model, treatment with a single dose I.V. of 4 mg/kg of mAb conjugated with Belotecan of established tumors led to a rapid and long-lasting regression.

More particularly, the inventors found that, in such configuration, an antibody-Belotecan conjugate is more efficient than a surrogate of Enfortumab vedotin, i.e., an Enfortumab vedotin product manufactured using non-GMP methods by a third party that is indistinguishable from the commercially available Enfortumab vedotin.

### ANTIBODIES

The antibodies in the conjugates of the invention are monoclonal antibodies (mAb) or monoclonal antibody fragments characterized by a specific amino acid sequence. If not indicated differently, the term "monoclonal" refers to a single species, i.e., single amino acid composition of antibodies or antibody fragments.

The antibodies provided herein show preferably specific binding to Nectin-4 and essentially no or no cross-reactivity to other proteins of the human Nectin-family, in particular Nectin-1, and/or rodent Nectin-4, such as Nectin-4 from rat and/or mouse.

The antigen-binding site of an inventive antibody comprises heavy chain variable domains/regions (VH) and/or antibody light chain variable domains/regions (VL), or pairs of VH/VL.

The term "antigen-binding site" denotes the region(s) of an antibody molecule to which a ligand (e.g., the antigen, i.e., Nectin-4, or antigen fragment of it) actually binds and which is derived from an antibody.

The variable domains/region denote each of the pair of light and heavy chains, which is involved directly in binding the antibody to the antigen. The variable domain of a heavy chain is abbreviated as "VH" and the variable domain of a light chain is abbreviated as "VL".

An antigen-binding site of an antibody according to the invention can contain six complementarity determining regions (CDRs) which contribute in varying degrees to the affinity of the binding site for the antigen. There are three heavy chain variable domain CDRs (CDR-H1, CDR-H2 and CDR-H3) and three light chain variable domain CDRs (CDR-L1, CDR-L2 and CDR-L3). Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

According to the present invention, a VH region or the CDRs thereof alone may constitute a complete antigen-binding site. In certain embodiments, the antibody comprises a VH region or the CDRs thereof as defined herein alone. In other embodiments, the antibody comprises a VH region or the CDRs thereof as defined herein together with a VL region or the CDRs thereof, particularly with a VL region or the CDRs thereof as defined herein.

The position of CDRs within a VH or VL region may be defined according to Kabat, et al., Sequences of Proteins of Immunological Interest, 5th Ed. Public Health Service, National Institutes of Health, Bethesda, MD (1991) or the IMGT numbering system, both of which are known to the person skilled in the art. The IMGT numbering has been defined to compare the variable domains whatever the antigen receptor, the chain type, or the species (Lefranc M.-P., "Unique database numbering system for immunogenetic analysis" Immunology Today, 18, 509 (1997); Lefranc M.-P., "The IMGT unique numbering for Immunoglobulins, T cell receptors and Ig-like domains" The Immunologist, 7, 132-136 (1999)). If not stated otherwise, the Kabat system is used herein.

As used herein, the terms "binding" and "specific binding" refer to the binding of the inventive antibody or fragment thereof to an epitope of the Nectin-4 antigen. The measure of the binding strength of an antibody is referred to as affinity. Methods for determining such a binding and/or affinity using in vitro assays are known to the person skilled in the art. According to the present invention, detection with flow cytometry, immuno-histochemistry and/or fluorescence are described and particularly preferred herein.

The affinity of the binding of an antibody to an antigen is defined by the terms Ka (rate constant for the association of the antibody from the antibody/antigen complex), KD (dissociation constant), and K_{dis} (KD/Ka).

In certain embodiments, the antibody of the conjugate of the invention may be a chimeric antibody, a multispecific antibody, in particular a bispecific antibody, a human antibody, a humanized antibody, or an antigen-binding fragment thereof.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" which binds two different epitopes.

In preferred embodiments, the antibody of the invention is a humanized antibody.

The term "humanized antibody" or "humanized version of an antibody" refers to antibodies for which both heavy and light chains are humanized as a result of antibody engineering. A humanized chain is typically a chain in which the V-region amino acid sequence has been changed so that, analyzed as a whole, is closer in homology to a human germline sequence than to the germline sequence of the species of origin. For example, a murine CDR may be grafted into the framework region of a human antibody to prepare the "humanized antibody." See, e.g., Riechmann, L., et al., Nature 332 (1988) 323-327; and Neuberger, M. S., et al., Nature 314 (1985) 268-270. Other forms of humanized antibodies encompassed by the present invention are those in which the constant region has been additionally modified or changed from that of the original antibody to generate the properties according to the invention. Humanization assessment is based on the resulting amino acid sequence and not on the methodology per se.

Another preferred embodiment refers to conjugates comprising human antibodies.

The term "human antibody", as used herein, is intended to include antibodies having variable and constant regions derived from human germ line immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production.

The antibody of the conjugate of the present invention may be of any suitable class. The term "class" refers to the type of constant domain or constant region possessed by its heavy chain. As used herein, "constant domain" or "constant region" denotes the sum of the domains of an antibody other than the variable region. The constant region is not directly involved in binding of an antigen but exhibits various effector functions. The antibody may be of any of the five major classes of antibodies, particularly of the five major classes of human antibodies: IgA, IgD, IgE, IgG, and IgM, or any subclass thereof (isotype), e.g., IgG1, IgG2, IgG3, IgG4, IgA1, and IgA2. The heavy chain constant domains that correspond to the different classes of immunoglobulins are called a, δ, ε, γ and µ, respectively. According to the present invention, an antibody of the class, particularly of the human class IgG, IgA or IgM or a fragment thereof is particularly suitable.

According to a preferred embodiment, an antibody of the conjugate of the invention is selected from class IgG, particularly from the class of human IgG, e.g., of subclass IgG1, IgG2, IgG3 of IgG4, of class IgM, of class IgA or an antigen-binding fragment thereof.

In certain embodiments, the antibody comprises a constant domain, particularly a heavy chain constant domain, more particularly a heavy chain constant domain of the class IgG, particularly of the class human IgG, e.g., of subclass IgG1, IgG2, IgG3 of IgG4, of class IgM, of class IgA, which has a reduced effector function compared to a wild-type sequence of the same subclass, e.g., which has a reduced binding to the Fc receptor. Examples of heavy chain constant domains with reduced effector functions may contain at least one of the mutations D265C, L234A, L235A, P331S, L234F and L235E of human IgG, e.g., IgG1 or IgG4 sequences.

An "antigen-binding fragment" of an antibody refers to a molecule comprising a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multi-specific antibodies formed from antibody fragments. The term also encompasses a fusion protein, e.g., a fusion protein with a non-immunoglobulin peptide or polypeptide, and a conjugate with a non-proteinaceous structure, e.g., a label or a toxin. The terms "antigen-binding fragment of an antibody (thereof)" and "fragment of an antibody (thereof)" may be used interchangeably herein.

The antibody or the antigen-binding fragment may be mono- or multivalent, i.e., it may comprise a single antigen-binding site or multiple antigen-binding sites. For example, Fab fragments have single antigen-binding site, antibodies of the IgG class or Fv or scFv fragments have two antigen-binding sites and antibodies of the IgM class have 5 antigen-binding sites. The term "antibody" also encompasses hetero-specific antibodies, e.g., hetero-bispecific antibodies, which have different antigen-binding sites, particularly antibodies, which are directed to two different epitopes on the antigen. As used herein, "epitope" is a region of an antigen that is bound by an antibody. The term "epitope" includes any polypeptide determinant capable of specific binding to an antibody.

### TUMOR-SELECTIVE ANTI-NECTIN-4 ANTIBODIES

In a particular embodiment, the antibody of the conjugate is a tumor-selective anti-Nectin-4 antibody, which specifically binds to Nectin-4 expressed by tumors with a higher affinity in comparison to Nectin-4 expressed by human keratinocytes, e.g., in vitro differentiated Nectin-4 expressing keratinocytes, e.g., a monoclonal antibody derived from the monoclonal antibody (mAb) 15A7.5, 9A2.7, 3A1.4 and/or 8F06. The mAb 15A7.5 is in particular preferred.

For comparison, the binding affinity may be detected by flow cytometry, immuno-histochemistry and/or fluorescence. Such specific binding allows, inter alia, less side effects during a treatment based on the inventive antibodies.

Accordingly, an antibody-drug conjugate comprising antibody 15A7.5, 9A2.7, 3A1.4 and/or 8F06 and in particular a humanized variant derived therefrom, represents a new way to improve therapeutic index of Nectin-4 positive cancer treatment through lower associated skin toxicity and higher anti-tumor selectivity and efficacy.

Tumor-selective anti-Nectin-4 antibodies and antigen-binding fragments thereof preferably show a dissociation constant KD of at least 40, preferably at least 45, more preferably at least 50 and most preferably at least 55 (nM).

Beside a lower binding affinity, the tumor-selective antibodies and fragments provided herein show also preferably lower internalization and/or cytotoxic activity towards keratinocytes in comparison to tumor cells.

According to an especially preferred embodiment, the tumor-selective antibodies and fragments show lower binding affinity, lower internalization, and lower cytotoxic activity towards keratinocytes in comparison to tumor cells.

Of course, a specific binding to Nectin-4 expressed by tumors is preferred.

According to a further aspect, the present invention also relates to an antibody-drug conjugate comprising a monoclonal antibody or antigen-binding fragment thereof characterized by binding to Nectin-4 expressed by tumors with a higher affinity in comparison to Nectin-4 expressed by human keratinocytes. For comparison, the binding affinity may be detected by flow cytometry, immuno-histochemistry and/or fluorescence.

In certain embodiments, the tumor-selective monoclonal ant-Nectin-4 antibody or antigen-binding fragment thereof comprises
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO: 1,7, 88, 96, or 104,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO: 2, 8, 88, 97 or 105,
      or an amino acid sequence comprising a substitution or insertion, particularly a conservative substitution and/or histidine insertion at least one amino acid, e.g., 1 or 2 amino acids,
   (iii) the CDR-H3 comprises an amino acid sequence according to SEQ ID NO: 3,9, 90, 98 or 106,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO: 4,10, 92, 100 or 108,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO: 5,11, 93, 101 or 109,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO: 6,12, 94, 102 or 110,
      or an amino acid sequence comprising a substitution or insertion, particularly a conservative substitution and/or histidine insertion of at least one amino acid, e.g., 1 or 2 amino acids.

SEQ ID NO: 1-6 define the six CDR sequences of the inventive parental antibody of 15A7.5 according to the IMGT numbering system. SEQ ID NO: 7-12 define the six CDR sequences of the inventive parental antibody according to Kabat.

Specific amino acid sequences for the SEQ ID NOs used herein are provided in the attached sequence listing.
SEQ ID NO:s 88-95 characterize mAb 9A2.7.
SEQ ID NO:s 96-103 characterize mAb 3A1.4.
SEQ ID NO:s 104-111 characterize mAb 8F06.

According to the present invention, a substitution or insertion of at least one amino acid, e.g., 1 or 2 amino acids in these CDR sequences is possible. In particular embodiments, a conservative amino acid substitution is preferable, i.e. a substitution of an amino acid by another amino acid with similar biochemical properties, for example a substitution of an aliphatic amino acid, e.g., Gly, Ala, Val, Leu or Ile, for another aliphatic amino acid, a basic amino acid, e.g., His, Lys or Arg, against another basic amino acid, an acidic amino acid or an amide thereof, e.g., Asp, Glu, Asn or Gln, against another acidic amino acid or an amide thereof, an aromatic amino acid, e.g., Phe, Tyr or Trp, against another aromatic amino acid, or a hydroxy or sulfur containing amino acid, e.g., Ser, Thr, Met or Cys, against another hydroxy or sulfur containing amino acid. In further particular embodiments, at least one amino acid, e.g., 1 or 2 histidine residues are inserted into one or more CDR sequences, e.g., CDR1, CDR2 or CDR3 of the heavy chain or the light chain. This might result in a preferential binding at a low pH, e.g., pH between 5.5 to 6.5 versus a neutral pH, e.g., pH 7.0 to 7.5.

According to a further preferred embodiment, the antibody or antigen-binding fragment thereof comprises according to the IMGT numbering system
(i) a VH region comprising the CDR-H1 of SEQ ID NO:1, the CDR-H2 of SEQ ID NO:2, and the CDR-H3 of SEQ ID NO:3,
   and optionally
(ii) a VL region comprising the CDR-L1 of SEQ ID NO:4, the CDR-L2 of the SEQ ID NO:5 and the CDR-L3 of the SEQ ID NO:6.

According to another preferred embodiment, the antibody or antigen-binding fragment thereof comprises according to Kabat
(i) a VH region comprising the CDR-H1 of SEQ ID NO:7, the CDR-H2 of SEQ ID NO:8, and the CDR-H3 of SEQ ID NO:9,
   and optionally
(ii) a VL region comprising the CDR-L1 of SEQ ID NO:10, the CDR-L2 of the SEQ ID NO:11 and the CDR-L3 of the SEQ ID NO:12.

According to another preferred embodiment, the antibody or antigen-binding fragment thereof comprises
(i) a VH region comprising the CDR-H1 of SEQ ID NO:88, the CDR- of SEQ ID NO:89, and the CDR-H3 of SEQ ID NO:90,
   and optionally
(ii) a VL region comprising the CDR-L1 of SEQ ID NO:92, the CDR-L2 of the SEQ ID NO:93 and the CDR-L3 of the SEQ ID NO:94.

According to another preferred embodiment, the antibody or antigen-binding fragment thereof comprises
(i) a VH region comprising the CDR-H1 of SEQ ID NO:96, the CDR- of SEQ ID NO:97, and the CDR-H3 of SEQ ID NO:98,
   and optionally
(ii) a VL region comprising the CDR-L1 of SEQ ID NO:100, the CDR-L2 of the SEQ ID NO:101 and the CDR-L3 of the SEQ ID NO:102.

According to another preferred embodiment, the antibody or antigen-binding fragment thereof comprises
(i) a VH region comprising the CDR-H1 of SEQ ID NO:104, the CDR-H2 of SEQ ID NO:105, and the CDR-H3 of SEQ ID NO:106,
   and optionally
(ii) a VL region comprising the CDR-L1 of SEQ ID NO:108, the CDR-L2 of the SEQ ID NO:109 and the CDR-L3 of the SEQ ID NO:110.

More particularly, the antibody or antigen-binding fragment thereof may comprise
(a) a VH region comprising an amino acid sequence according to SEQ ID NO:13, 91, 99 or 107 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:14, 95, 103 or 111 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:13 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:14 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:91 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:95 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:99 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:103 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:107 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:111 or an amino sequence having an identity thereof of at least 85%, at least 90%, at least 95% or at least 99% over the whole length of the sequence.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, Alignment for purposes of determining percent amino acid sequence identity can be achieved in various ways that are within the skill in the art, for instance, using publicly available computer software such as BLAST.

In particular embodiments, the inventive humanized or human antibodies are defined by combination of at least 3, preferably 6, complementarity-determining regions (CDRs), i.e., relate to antibodies or antigen-binding fragments thereof comprising
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO:21, 35, 49 or 63,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO:22, 36, 50 or 64,
      or an amino acid sequence comprising a substitution or insertion, particularly a conservative substitution and/or histidine insertion of at least one amino acid, e.g., 1 or 2 amino acids,
   (iii) the CDR-H3 comprises an amino acid sequence according to SEQ ID NO:23, 37, 51 or 65,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:24, 38, 52 or 66,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO:25, 39, 53 or 67,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO:26, 40, 54 or 68,
      or an amino acid sequence comprising a substitution or insertion, particularly a conservative substitution and/or histidine insertion of at least one amino acid, e.g., 1 or 2 amino acids.

Specific humanized or human antibodies according to the present invention may comprise
(a)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:24, the CDR-L2 of the SEQ ID NO:25, and the CDR-L3 of the SEQ ID NO:26, or
(b)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:38, the CDR-L2 of the SEQ ID NO:39, and the CDR-L3 of the SEQ ID NO:40, or
(c)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:52, the CDR-L2 of the SEQ ID NO:53, and the CDR-L3 of the SEQ ID NO:54, or
(d)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:66, the CDR-L2 of the SEQ ID NO:67, and the CDR-L3 of the SEQ ID NO:68,
      or
(e)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:38, the CDR-L2 of the SEQ ID NO:39, and the CDR-L3 of the SEQ ID NO:40,
      or
(f)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:52, the CDR-L2 of the SEQ ID NO:5, and the CDR-L3 of the SEQ ID NO:54,
      or
(g)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:66, the CDR-L2 of the SEQ ID NO:67, and the CDR-L3 of the SEQ ID NO:68,
      or
(h)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:24, the CDR-L2 of the SEQ ID NO:25, and the CDR-L3 of the SEQ ID NO:26,
      or
(j)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:52, the CDR-L2 of the SEQ ID NO:53, and the CDR-L3 of the SEQ ID NO:54,
      or
(k)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:66, the CDR-L2 of the SEQ ID NO:67, and the CDR-L3 of the SEQ ID NO:68,
      or
(I)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:24, the CDR-L2 of the SEQ ID NO:25, and the CDR-L3 of the SEQ ID NO:26,
      or
(m)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:38, the CDR-L2 of the SEQ ID NO:39, and the CDR-L3 of the SEQ ID NO:40,
      or
(n)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:66, the CDR-L2 of the SEQ ID NO:67, and the CDR-L3 of the SEQ ID NO:68,
      or
(o)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:24, the CDR-L2 of the SEQ ID NO:25, and the CDR-L3 of the SEQ ID NO:26,
      or
(p)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:38, the CDR-L2 of the SEQ ID NO:39, and the CDR-L3 of the SEQ ID NO:40,
      or
(q)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:52, the CDR-L2 of the SEQ ID NO:53, and the CDR-L3 of the SEQ ID NO:54.

In particular preferred are the humanized antibodies (a), (b), (c), (d), (j), (k), (n) and (q) as defined above.

In particular embodiments, the inventive humanized or human antibodies are defined by combination of at least 3, preferably 6, complementarity-determining regions (CDRs), i.e., relate to antibodies or antigen-binding fragments thereof comprising
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO:21, 35, 49 or 63,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO:22, 36, 50 or 64
      or an amino acid sequence comprising a substitution or insertion, particularly a conservative substitution and/or histidine insertion of at least one amino acid, e.g., 1 or 2 amino acids,
   (iii) the CDR-H3 comprises an amino acid sequence according to SEQ ID NO:23, 37, 51 or 65,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:24, 38, 52 or 66,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO:25, 39, 53 or 67,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO:26, 40, 54 or 68,
      or an amino acid sequence comprising a substitution or insertion, particularly a conservative substitution and/or histidine insertion of at least one amino acid, e.g., 1 or 2 amino acids

Specific humanized or human antibodies according to the present invention may comprise
(a)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:24, the CDR-L2 of the SEQ ID NO:25, and the CDR-L3 of the SEQ ID NO:26, or
(b)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:38, the CDR-L2 of the SEQ ID NO:39, and the CDR-L3 of the SEQ ID NO:40, or
(c)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:52, the CDR-L2 of the SEQ ID NO:53, and the CDR-L3 of the SEQ ID NO:54, or
(d)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:66, the CDR-L2 of the SEQ ID NO:67, and the CDR-L3 of the SEQ ID NO:68,
      or
(e)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:38, the CDR-L2 of the SEQ ID NO:39, and the CDR-L3 of the SEQ ID NO:40,
      or
(f)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:52, the CDR-L2 of the SEQ ID NO:53, and the CDR-L3 of the SEQ ID NO:54,
      or
(g)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:66, the CDR-L2 of the SEQ ID NO:67, and the CDR-L3 of the SEQ ID NO:68,
      or
(h)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:24, the CDR-L2 of the SEQ ID NO:25, and the CDR-L3 of the SEQ ID NO:26,
      or
(j)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:52, the CDR-L2 of the SEQ ID NO:53, and the CDR-L3 of the SEQ ID NO:54,
      or
(k)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:66, the CDR-L2 of the SEQ ID NO:67, and the CDR-L3 of the SEQ ID NO:68,
      or
(I)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:24, the CDR-L2 of the SEQ ID NO:25, and the CDR-L3 of the SEQ ID NO:26,
      or
(m)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:38, the CDR-L2 of the SEQ ID NO:39, and the CDR-L3 of the SEQ ID NO:40,
      or
(n)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:66, the CDR-L2 of the SEQ ID NO:67, and the CDR-L3 of the SEQ ID NO:68,
      or
(o)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:24, the CDR-L2 of the SEQ ID NO:25, and the CDR-L3 of the SEQ ID NO:26,
      or
(p)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:38, the CDR-L2 of the SEQ ID NO:39, and the CDR-L3 of the SEQ ID NO:40,
      or
(q)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:52, the CDR-L2 of the SEQ ID NO:53, and the CDR-L3 of the SEQ ID NO:54.

Particularly preferred are the humanized antibodies (a), (b), (c), (d), (j), (k), (n) and (q) as defined above.

Of course, the humanized or human antibodies according to the invention may be also defined by their VH and/or VL regions.

Such antibodies may comprise
(i) a VH region comprising an amino acid sequence selected from SEQ ID NO:27, 41, 55 or 69 or an amino acid sequence having an identity thereof of at least 85%, at least 90%, at least 95% or at least 99%,
   and optionally
(ii) a VL region, particularly a VL region comprising an amino acid sequence selected from SEQ ID NO:28, 42, 56, 70 or 71 or an amino sequence having an identity thereof of at least 85%, at least 90%, at least 95% or at least 99%.

Specific humanized or human antibodies according to the invention may be defined by their VH and/or VL regions, wherein such antibodies comprise
(a)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(b)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(c)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%,
      or
(d)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(e)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(f)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%,
      or
(g)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(h)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(j)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%,
      or
(k)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(I)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(m)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(n)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(o)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(p)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(q)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%.

In particular preferred are the humanized antibodies (a), (b), (c), (d), (j), (k), (n) and (q) as defined above.

### FURTHER ANTI-NECTIN-4 ANTIBODIES

In a further embodiment, the antibody of the conjugate of the invention is the antibody 5A12.2 or an antigen-binding fragment thereof.

SEQ ID NO:s 80-87 characterize mAb 5A12.2.

According to a preferred embodiment, the 5A12.2 antibody or antigen-binding fragment thereof comprises
(i) a VH region comprising the CDR-H1 of SEQ ID NO:80, the CDR- of SEQ ID NO:81, and the CDR-H3 of SEQ ID NO:82,
   and optionally
(ii) a VL region comprising the CDR-L1 of SEQ ID NO:84, the CDR-L2 of the SEQ ID NO:85 and the CDR-L3 of the SEQ ID NO:86.

According to another preferred embodiment, the 5A12.2 antibody or antigen-binding fragment thereof may comprise
(a) a VH region comprising an amino acid sequence according to
   SEQ ID NO:83 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:87 or an amino sequence having an identity thereof of at least 85%, at least 90%, at least 95% or at least 99% over the whole length of the sequence.

In a further embodiment, the antibody of the conjugate of the invention is the antibody HA22, which is the antibody of the marketed antibody-drug conjugate Enfortumab vedotin, or a HA22-derived antibody.

HA22 is a human monoclonal anti-Nectin-4 antibody described in WO2012/047724, the content of which is incorporated herein by reference. A HA22 antibody or a HA22-derived antibody particularly refers to an IgG1 antibody comprising a heavy chain G1m17 or G1m3 haplotype sequence optionally with a constant region having a reduced effector function as described above, e.g., the TM mutation: P331S, L234F and L235E, or the LALA mutation: L234A and L235A and a kappa light chain sequence. In particular, the HA22 VH sequence and its human IgG1 constant region is shown in Fig. 3A, and the HA22 VL sequence and its human kappa constant region is shown in Fig. 3B of WO 2012/047724.

In certain embodiments, a HA22-derived monoclonal anti-Nectin-4 antibody or antigen-binding fragment thereof is defined by its CDR sequences and comprises
(a) a variable heavy chain (VH) region
   comprising complementarity-determining regions (CDRs) CDR-H1, CDR-H2 and CDR-H3, wherein
   (i) the CDR-H1 comprises an amino acid sequence according to SEQ ID NO:72,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO:73,
      or an amino acid sequence comprising a substitution or insertion, particularly a conservative substitution and/or histidine insertion of at least one amino acid, e.g., 1 or 2 amino acids,
   (iii) the CDR-H3 comprises an amino acid sequence according to SEQ ID NO:74,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:75,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO:76,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO:77,
      or an amino acid sequence comprising a substitution or insertion, particularly a conservative substitution and/or histidine insertion of at least one amino acid, e.g., 1 or 2 amino acids.

SEQ ID NO: 72-77 define the six CDR sequences of the parental antibody HA22 according to the IMGT numbering system.

In particular embodiments, a HA22 derived antibody may be defined by its VH and/or VL regions, wherein such antibody comprises
(a)
   (i) a VH region comprising an amino acid sequence according to SEQ ID NO:78 or an amino acid sequence having an identity thereof of at least 85%, at least 90%, at least 95% or at least 99%,
      and optionally
   (ii) a VL region, particularly a VL region comprising an amino acid sequence according to SEQ ID NO:79 or an amino sequence having an identity thereof of at least 85%, at least 90%, at least 95% or at least 99%.

In even more particular embodiments, a HA22 derived antibody comprises
(a)
   (i) a VH region comprising an amino acid sequence according to SEQ ID NO:78,
      and
   (ii) a VL region comprising an amino acid sequence according to SEQ ID NO:79.

### BELOTECAN AND DERIVATIVES

The drug D of the antibody conjugate of the invention is Belotecan or a derivative thereof represented by the formula (II) 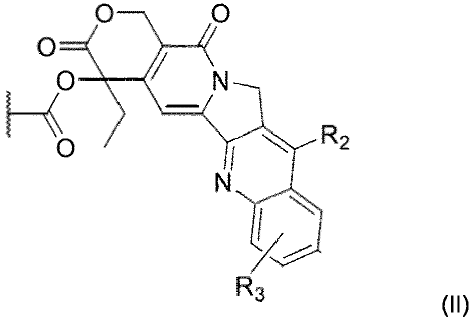 wherein
- R²: is -(CH₂)₂NR¹CH(CH₃)₂,
- R¹: is H or -SO₂CH₃, and
- R³: is H, amino, or C₁-C₆ alkyl, preferably H.

The position where M or L is attached to Belotecan or the derivative thereof is indicated by the waved line. Preferably R¹ is H.

### CONJUGATION

The Belotecan or Belotecan derivative D is covalently conjugated to the antibody or antigen-binding fragment thereof by linker M.

M may be cleavable under physiological conditions, e.g., by physiological enzymes. Specific examples of cleavable linkers are peptide-based linkers, which may be subject to cleavage by a protease, or glycoside-based linkers, which may be subject to cleavage by a glycosidase.

In certain embodiments, the linker M is a hydrophilic polysarcosine linker e.g., as described by Conilh et at. "Exatecan antibody drug conjugates based on a hydrophilic polysarcosine drug-linker platform" (Pharmaceuticals 14 (2021), 247). Further preferred linkers include linkers comprising at least one ethylene glycol unit, e.g., 1, 2, 3, 4, 5, 6, 7, 8, 9, or 10 or more ethylene glycol units, e.g., the linker of the antibody-drug conjugate MEDI7247, an mcc-triazole spacer-PEG7-x-Lys-PABC glycol linker from Trodelvy^{®}, Further preferred linkers include oligopeptide, particularly di- to decapeptide, e.g., tetrapeptide sequences such as glycine-glycine-phenylalanine-glycine from Enhertu^{®} or valine-alanine. Further preferred linkers include highly polar spacers such as an acyl group, carbamoyl group and/or sulfamide group added to at least at least one ethylene glycol unit, e.g., 1, 2, 3, 4, 5, 6, 7, 8, 9, or 10 or more ethylene glycol units, e.g., the linker technology called Hydraspace^{™}.

In particular embodiments, the linker M of the inventive antibody drug conjugate comprises an oligopeptide, particularly di- to decapeptide, e.g. Val-Ala or GGFG (Gly-Gly-Phe-Gly) sequence and optionally at least one ethylene glycol unit, e.g., 1, 2, 3, 4, 5, 6, 7, 8, 9, or 10 or more ethylene glycol units. A preferred linker according to this aspect is a Val-Ala or GGFG linker with 8 ethylene glycol units.

In certain embodiments, the linker M is a hydrophilic polysarcosine linker comprising, e.g., up to 15 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase.

In certain embodiments, the linker M is a hydrophilic polysarcosine linker comprising, e.g., about 8-12 sarcosine units, and at least one ethylene glycol unit, e.g., up to 10 ethylene glycol units, and wherein the linker is subject to cleavage by a glycosidase, and particularly subject to cleavage by a glucuronidase.

In certain embodiments, the linker M is a hydrophilic polysarcosine linker comprising 10 sarcosine units, and 2 ethylene glycol units, and wherein the linker is subject to cleavage by a glucuronidase.

A further aspect of the invention relates to a linker-drug conjugate comprising:
(i) a hydrophilic polysarcosine linker comprising, e.g., about 8-12 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase, and wherein the linker comprises a thiol-reactive group, e.g., a maleimide group capable of reacting with cysteine residues on an antibody or antigen-binding fragment thereof, and
(ii) Belotecan or a derivative thereof covalently attached to the linker.

In particular embodiments, the linker-drug conjugate comprises:
(i) a hydrophilic polysarcosine linker comprising 10 sarcosine units, and 2 ethylene glycol units, wherein the linker is subject to cleavage by a glycosidase, and particularly subject to cleavage by a glucuronidase, and wherein the linker comprises a maleimide group capable of reacting with cysteine residues on an antibody or antigen-binding fragment thereof, and
(ii) Belotecan or a derivative thereof covalently attached to the linker.

According to other embodiments, the linker M of the inventive antibody drug conjugate may be characterized by formula (III):

-L-A-E- (III),

wherein
- L: is -(C₁-C₆-alkylene)-(O-CH₂)ₘ-NR¹-,
-(C₁-C₆-alkylene)-NR¹-, or
-(C₁-C₅-alkylene)-NR²C(C=O)-(C₁-C₅-alyklene)-(O-CH₂)^{m}-NR¹-,
wherein R¹ and R² are each independently -H, C₁-C₃-alkyl, in particular methyl, or benzyl,
m is an integer from 1 to 6, in particular 1,
- A: is a peptide comprising 2 to 8 amino acids, preferably 2 to 4 amino acids, wherein A is substituted with at least one polyol, wherein polyol is -(C₁-C₆ alkylene)-X^{a}-Y^{b}, wherein:
X^{a} is -NR^{c}C(=O)- or -C(=O)NR^{c}-;
Y^{b} is -CH₂-(C*₁-C*₅-alkyl)-, each C* substituted with a OH group,
- R^{c}: is -H, C₁-C₆-alkyl, C₁-C₆ fluoroalkyl, C₃-C₆ cycloalkyl, aryl, heteroaryl, or benzyl, and
- E: is -C(=O)-(C₁-C₁₀-alkylene)-Q-, wherein Q is an antibody coupling group.
- L: is -(C₁-C₆-alkylene)-(O-CH₂)ₘ-NR¹-, in particular -(C₁-C₄-alkylene)-O-CH₂-NR¹-, -(C₁-C₆-alkylene)-NR¹-, in particular -(C₁-C₄-alkylene)-NR¹-, or-(C₁-C₅-alkylene)-NR²C(C=O)-(C₁-C₅-alyklene)-(O-CH₂)ₘ-NR¹-, in particular -(C₁-C₅-alkylene)-NR²C(C=O)-(C₁-C₅-alyklene)-O-CH₂-NR¹-, wherein R¹ and R² are each independently -H, C₁-C₃-alkyl, in particular methyl, or benzyl, and m is an integer from 1 to 6, in particular 1.

According to a preferred embodiment L is -(C₁-C₄-alkylene)-O-CH₂-NR¹-, -(C₁-C₄-alkylene)-NR¹-, or -(C₁-C₅-alkylene)-NR²C(C=O)-(C₁-C₅-alyklene)-O-CH₂-NR¹-, wherein R¹ and R² are each independently -H, methyl, or benzyl.
- A: is a peptide comprising 2 to 8 amino acids, preferably 2 to 4 amino acids, wherein A is substituted with at least one polyol, wherein polyol is -(C₁-C₆ alkylene)-X^{a}-Y^{b}, wherein:
X^{a} is -NR^{c}C(=O)- or -C(=O)NR^{c}-;
Y^{b} is -CH₂-(C*₁-C*₅-alkyl)-, each C* substituted with a OH group,
- R^{c}: is -H, C₁-C₆-alkyl, C₁-C₆ fluoroalkyl, C₃-C₆ cycloalkyl, aryl, heteroaryl, or benzyl.

As used herein "alkylene" refers to a saturated linear or branched divalent hydrocarbon radical.

As used herein "cycloalkyl" refers to the radical of a saturated carbocyclic ring. Suitable cycloalkyls include cyclohexyl, cyclopentyl, cyclobutyl and cyclopropyl.

The term "aryl" as used herein, includes substituted or unsubstituted single-ring aromatic groups in which each atom of the ring is carbon. "Aryl" groups include, but are not limited to, phenyl, phenol, aniline, etc. The terms "aryl" also includes "polycyclyl", "polycycle", and "polycyclic" ring systems having two or more rings in which two or more atoms are common to two adjoining rings, e.g., the rings are "fused rings," wherein at least one of the rings is aromatic, e.g., the other cyclic rings can be cycloalkyls, cycloalkenyls, cycloalkynyls, or aromatic rings.

Preferably, the ring is a 5- to 7-membered ring, more preferably a 6-membered ring. Aryl groups include, but are not limited to, phenyl, phenol, aniline, and the like.

"Heteroaryl" as used herein refers to substituted or unsubstituted aromatic single ring structures, preferably 6- to 18-member rings, preferably 5- to 7-membered rings, more preferably 5- to 6-membered rings, whose ring structures include at least one heteroatom (e.g., O, N, or S).

The term "heteroaryl" as used herein, refers to substituted or unsubstituted aromatic single ring structures, preferably 6- to 18-member rings, preferably 5- to 7-membered rings, more preferably 5- to 6-membered rings, whose ring structures include at least one heteroatom (e.g., O, N, or S), preferably one to four or one to three heteroatoms, more preferably one or two heteroatoms. When two or more heteroatoms are present in a heteroaryl ring, they may be the same or different. For examples, "heteroaryl" moieties include, but are not limited to, pyrrole, furan, thiophene, imidazole, oxazole, thiazole, pyrazole, pyridine, pyrazine, pyridazine, quinoline, pyrimidine, indolizine, indole, indazole, benzimidazole, benzothiazole, benzofuran, benzothiophene, cinnoline, phthalazine, quinazoline, carbazole, phenoxazine, quinoline, purine and the like.

Unless specifically stated as "unsubstituted," references to chemical moieties herein are understood to also include substituted variants, i.e. can be substituted with one or more (e.g., 2, 3, 4, 5, 6 or more) substituents. For example, reference to an "alkyl" group or moiety implicitly includes both substituted and unsubstituted variants. Examples of substituents on chemical moieties includes but is not limited to, halogen, hydroxyl, carbonyl (such as carboxyl, alkoxycarbonyl, formyl, or acyl), thiocarbonyl (such as thioester, thioacetate, or thioformate), alkoxyl, alkylthio, acyloxy, phosphoryl, phosphate, phosphonate, amino, amido, amidine, imine, cyano, nitro, azido, sulfhydryl, alkylthio, sulfate, sulfonate, sulfamoyl, sulfonamido, sulfonyl, heterocyclyl, aralkyl, or aryl or heteroaryl moiety.

The amino acids of peptide A may be selected independently. The amino acids may be independently L or D amino acids. According to one preferred embodiment at least one amino acid is an L amino acid. According to other preferred embodiments all amino acids are D or L amino acids. The amino acids alanine (Ala), valine (Val), or glycine (Gly) are in particular preferred.

Suitable examples for a peptide A comprise inter alia -Ala-Ala-, -Gly-Gly-, -Val-Val-, - Val-Ala-, -Ala-Ala-Ala-, -Gly-Ala-Gly-Gly-, -Gly-Gly-Ala-Gly-, -Gly-Val-Gly-Gly-, -Gly-Gly-Val-Gly-, -Gly-Gly-Phe-Gly-, or -Gly-Phe-Gly-Gly- but also flexible linkers like glycine polymers (G)ₙ, glycine-serine polymers (including, for example, (GS)ₙ, (GSGGS)ₙ and (GGGS)ₙ , where n is an integer of at least one, glycine-alanine polymers, alanine-serine polymers, and other flexible linkers known in the art. Such flexible linkers include, but are not limited to -Gly-Gly-Ser-Gly-, -Gly-Gly-Ser-Gly-Gly- , -Gly-Ser-Gly-Ser-Gly-, -Gly-Ser-Gly-Gly-Gly-, -Gly-Gly-Gly-Ser-Gly-, -Gly-Ser-Ser-Ser-Gly-, and the like.

E is -C(=O)-(C₁-C₁₀-alkylene)-Q-, wherein Q is an antibody coupling group. Such antibody coupling groups are well known to the person skilled in the art. Before being coupled, the antibody coupling group is preferably a maleimide group.

After being coupled Q is preferably 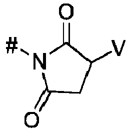 wherein
- #: is the site covalently attached to the remainder of E, i.e. -C(=O)-(C₁-C₁₀-alkylene)-#, and
- V: is as herein defined.

Corresponding coupling reactions are well known to the person skilled in the art.

According to a preferred embodiment, Y^{b} is Cs-alkyl substituted with 5 OH groups.

According to a further preferred embodiment the at least one polyol is 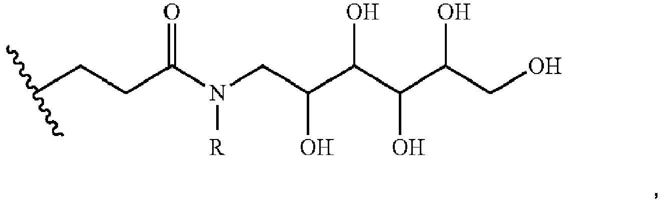 wherein R is H or methyl.

In certain embodiments, the antibody drug conjugate has a molar drug-antibody/antibody fragment ratio (DAR) of greater than 1, i.e., more than one drug molecule is attached to an antibody/antibody fragment. Typically, the conjugate has a DAR of about 2:1 to about 16:1, particularly of about 4:1 to about 10:1 and more particularly of about 6:1 to about 8:1. The DAR may be calculated from a statistical distribution according to known methods.

The linker-drug conjugates described herein are particularly suitable for attachment to an anti-Nectin-4 antibody as described herein. It should be noted, however, that the linker-drug conjugates are also suitable for attachment to any antibody or antigen-binding fragment thereof, e.g., an antibody binding to a tumor antigen or an antigen-binding fragment thereof.

The antibody drug conjugates of the present invention may be prepared by known methods.

In general, the antibody or the antigen-binding fragment thereof may be produced in a suitable host cell comprising a nucleic acid molecule, e.g., a DNA molecule, encoding an antibody VH region, or an antibody VL region, or encoding a complete antibody or an antibody fragment, or a vector or vector system, i.e. a plurality of vectors, comprising said nucleic acid molecule(s), preferably in operative linkage with an expression control sequence, particularly with a heterologous expression control sequence. The host cell may be any known host cell for producing antibodies or antibody fragments, e.g., a prokaryotic cell such as an E. coli cell, a yeast cell, an insect cell, or a mammalian cell, e.g., a CHO cell or a hybridoma cell.

The antibody or antigen-binding fragment thereof may be reacted with a linker-drug conjugate to obtain the antibody-drug conjugate. The linker-drug conjugate comprises a drug molecule, e.g., having attached thereto a suitable linker, wherein the linker comprises a reactive group capable of reacting with desired attachment positions on the antibody or antigen-binding fragment thereof. For attachment to cysteine residues, the linker-drug conjugate comprises a thiol-reactive group, e.g., a maleimide group.

### PHARMACEUTICAL COMPOSITIONS

A further aspect of the present invention is a pharmaceutical composition comprising an active agent, which is an antibody-drug conjugate as herein described, i.e. comprising a monoclonal antibody or an antigen-binding fragment thereof wherein the monoclonal antibody or the antigen-binding fragment thereof binds to Nectin-4 and wherein the drug is Belotecan or a derivative thereof as defined herein and a pharmaceutically acceptable carrier and/or excipient.

Examples of suitable carriers and excipients for formulating antibody drug conjugates include saline and aqueous buffer solutions and are well known in the art. Typically, the pharmaceutical composition is adapted for parenteral administration, e.g., for subcutaneous, intramuscular, or intravenous injection or by infusion. In further embodiments, the pharmaceutical composition is adapted for local administration, e.g., for intravesical instillation into the bladder.

Depending on the stage and the severity of the disorder, the pharmaceutical composition may be administered once or several times in a therapeutically effective dose to a subject in need thereof, particularly to a human subject. For example, it may be administered once or several times daily, each second day, two times weekly or weekly for a suitable period, e.g., of at least one week, or at least one month.

### MEDICAL APPLICATIONS

According to a further aspect of the invention, the antibody-drug conjugate or pharmaceutical composition as described above is used in medicine, including human and veterinary medicine, particularly in human medicine.

In a particular embodiment, the antibody-drug conjugate or pharmaceutical composition as described above is used in a method for the prevention and/or treatment of a Nectin-4 associated disorder such as cancer and/or an inflammatory disease, particularly for the prevention and/or treatment of a Nectin-4 positive cancer and/or inflammatory disease, more particularly for the prevention and/or treatment of a cancer and/or an inflammatory disease associated with Nectin-4 overexpression.

The cancer to be prevented and/or treated may be any kind of cancer, wherein the term "cancer" is used herein to refer to proliferative diseases. The cancer to be prevented and/or treated according to the present invention is preferably selected from the group consisting of bladder, urothelial, endometrial, cervical, colorectal, liver, thyroid, breast, pancreatic, lung, ovarian head and neck and/or esophagus cancer.

In therapeutic applications, the active agent is administered in an effective amount to a subject in need thereof, particularly to a human subject. The dose will depend on the specific type of agent, e.g., type of antibody or antibody fragment, the type of disease, and the mode of administration, e.g., locally or systemically.

In the prevention and/or treatment of cancer, a therapeutic dose of an antibody drug conjugate is typically from about 0.3 mg/kg to about 10 mg/kg.

The antibody drug conjugate may be administered alone or together with a further active agent, which may be selected from chemotherapeutic agents, e.g., antimetabolites, alkylating agents, intercalating agents, or anti-mitotic agents), inhibitors of specific kinases e.g., tyrosine kinase inhibitors, serine/threonine kinase inhibitors or phosphoinositide kinase inhibitors, immunotherapeutic compounds, e.g., immune checkpoint inhibitors, CAR-T cells, therapeutic vaccines or oncolytic viruses.

Sequence Listing Information:
        DTD Version: V1_3
        File Name: 203922pep gesamt.xml
        Software Name: WIPO Sequence
        Software Version: 2.1.0
        Production Date: 2022-07-20
General Information:
        Current application / Applicant file reference: 206764PEP
        Applicant name: Emergence Therapeutics AG et al
        Applicant name / Language: en
        Invention title: Novel Antibody-Drug conjugates comprising an
anti-Nectin-4 Antibody and Beleotecan or a derivative thereof ( en )
        Sequence Total Quantity: 123
Sequences:
        Sequence Number (ID): 1
        Length: 8
        Molecule Type: AA
        Features Location/Qualifiers:
                - REGION, 1..8
                        > note, CDR1 VH_15A7.5 IMGT
                - source, 1..8
                        > mol_type, protein
                        > organism, synthetic construct
        Residues:
        GFTFSNYG
8

        Sequence Number (ID): 2
        Length: 8
        Molecule Type: AA
        Features Location/Qualifiers:
                - REGION, 1..8
                        > note, CDR2 VH_15A7.5 IMGT
                - source, 1..8
                        > mol_type, protein
                        > organism, synthetic construct
        Residues:
        ISNLAYGI
8

        Sequence Number (ID): 3
        Length: 12
        Molecule Type: AA
        Features Location/Qualifiers:
                - REGION, 1..12
                        > note, CDR3 VH_15A7.5 IMGT
                - source, 1..12
                        > mol_type, protein
                        > organism, synthetic construct
        Residues:
        ARGARATGWF  AY
12

Sequence Number (ID): 4
Length: 6
Molecule Type: AA
Features Location/Qualifiers:
- REGION, 1..6
> note, CDR1 VK_15A7.5 IMGT
- source, 1..6
> mol_type, protein
> organism, synthetic construct
Residues:
QNVDTH
6

Sequence Number (ID): 5
Residues:
000

Sequence Number (ID): 6
Length: 9
Molecule Type: AA
Features Location/Qualifiers:
- REGION, 1..9
> note, CDR3 VK_15A7.5 IMGT
- source, 1..9
> mol_type, protein
> organism, synthetic construct
Residues:
QQYNSYPLT
9

Sequence Number (ID): 7
Length: 5
Molecule Type: AA
Features Location/Qualifiers:
- REGION, 1..5
> note, CDR1 VH_15A7.5 Kabat
- source, 1..5
> mol_type, protein
> organism, synthetic construct
Residues:
NYGMA
5

Sequence Number (ID): 8
Length: 17
Molecule Type: AA
Features Location/Qualifiers:
- REGION, 1..17
> note, CDR2 VH_15A7.5 Kabat
- source, 1..17

>                    > mol_type, protein
                    > organism, synthetic construct
        Residues:
        FISNLAYGIN  YADTVTG
17

        Sequence Number (ID): 9
        Length: 10
        Molecule Type: AA
        Features Location/Qualifiers:
                - REGION, 1..10
                        > note, CDR3 VH_15A7.5 Kabat
                - source, 1..10
                        > mol_type, protein
                        > organism, synthetic construct
        Residues:
        GARATGWFAY
10

        Sequence Number (ID): 10
        Length: 11
        Molecule Type: AA
        Features Location/Qualifiers:
                - REGION, 1..11
                        > note, CDR1 VK_15A7.5 Kabat
                - source, 1..11
                        > mol_type, protein
                        > organism, synthetic construct
        Residues:
        KASQNVDTHV  A
11

        Sequence Number (ID): 11
        Length: 7
        Molecule Type: AA
        Features Location/Qualifiers:
                - REGION, 1..7
                        > note, CDR2 VK_15A7.5 Kabat
                - source, 1..7
                        > mol_type, protein
                        > organism, synthetic construct
        Residues:
        SASYRYS
7

        Sequence Number (ID): 12
        Length: 9
        Molecule Type: AA
        Features Location/Qualifiers:
                - REGION, 1..9
                        > note, CDR3 VK_15A7.5 Kabat
                - source, 1..9

> mol_type, protein
> organism, synthetic construct
Residues:
QQYNSYPLT
9

Sequence Number (ID): 13
Length: 119
Molecule Type: AA
Features Location/Qualifiers:
- REGION, 1..119
> note, VH_15A7.5
- source, 1..119
> mol_type, protein
> organism, synthetic construct
Residues:
EVKLVESGGG LVQPGGSRKL SCAASGFTFS NYGMAWVRQA PGKGPEWVAF ISNLAYGINY
60
ADTVTGRFTI SRENAKNTLY LEMRSLRSED TAMYYCARGA RATGWFAYWG QGTLVTVSA
119

Sequence Number (ID): 14
Length: 107
Molecule Type: AA
Features Location/Qualifiers:
- REGION, 1..107
> note, VK_15A7.5
- source, 1..107
> mol_type, protein
> organism, synthetic construct
Residues:
DIVMTQSQKF MSTSIGDRVS VTCKASQNVD THVAWYQEKP GQSPKALIYS ASYRYSGVPD
60
RFTGSGSGTD FTLTISNVQS EDLADYFCQQ YNSYPLTFGG GTKLEIK
107

Sequence Number (ID): 15
Length: 9
Molecule Type: AA
Features Location/Qualifiers:
- REGION, 1..9
> note, CDR1 H0_15A7.5IMGT
- source, 1..9
> mol_type, protein
> organism, synthetic construct
Residues:
GFTFSNYGM
9

Sequence Number (ID): 16
Length: 8
Molecule Type: AA

Features Location/Qualifiers:
- REGION, 1..8
> note, CDR2 H0_15A7.5IMGT
- source, 1..8
> mol_type, protein
> organism, synthetic construct
Residues:
ISNLAYGI
8

Sequence Number (ID): 17
Length: 12
Molecule Type: AA
Features Location/Qualifiers:
- REGION, 1..12
> note, CDR3 H0_15A7.5IMGT
- source, 1..12
> mol_type, protein
> organism, synthetic construct
Residues:
ARGARATGWF AY
12

Sequence Number (ID): 18
Length: 6
Molecule Type: AA
Features Location/Qualifiers:
- REGION, 1..6
> note, CDR1 L0_15A7.5 IMGT
- source, 1..6
> mol_type, protein
> organism, synthetic construct
Residues:
QNVDTH
6

Sequence Number (ID): 19
Residues:
000

Sequence Number (ID): 20
Length: 9
Molecule Type: AA
Features Location/Qualifiers:
- REGION, 1..9
> note, CDR3 L0_15A7.5 IMGT
- source, 1..9
> mol_type, protein
> organism, synthetic construct
Residues:
QQYNSYPLT

9

Sequence Number (ID): 21
Length: 5
Molecule Type: AA
Features Location/Qualifiers:
- REGION, 1..5
> note, CDR1 H0_15A7.5Kabat
- source, 1..5
> mol_type, protein
> organism, synthetic construct
Residues:
NYGMN
5

Sequence Number (ID): 22
Length: 17
Molecule Type: AA
Features Location/Qualifiers:
- REGION, 1..17
> note, CDR2 H0_15A7.5Kabat
- source, 1..17
> mol_type, protein
> organism, synthetic construct
Residues:
FISNLAYGIN  YADSVKG
17

Sequence Number (ID): 23
Length: 10
Molecule Type: AA
Features Location/Qualifiers:
- REGION, 1..10
> note, CDR3 H0_15A7.5Kabat
- source, 1..10
> mol_type, protein
> organism, synthetic construct
Residues:
GARATGWFAY
10

Sequence Number (ID): 24
Length: 11
Molecule Type: AA
Features Location/Qualifiers:
- REGION, 1..11
> note, CDR1 L0_15A7.5 Kabat
- source, 1..11
> mol_type, protein
> organism, synthetic construct
Residues:
RASQNVDTHV  A

11

Sequence Number (ID): 25
Length: 7
Molecule Type: AA
Features Location/Qualifiers:
- REGION, 1..7
> note, CDR2 L0_15A7.5 Kabat
- source, 1..7
> mol_type, protein
> organism, synthetic construct
Residues:
SASYLQS
7

Sequence Number (ID): 26
Length: 9
Molecule Type: AA
Features Location/Qualifiers:
- REGION, 1..9
> note, CDR3 L0_15A7.5 Kabat
- source, 1..9
> mol_type, protein
> organism, synthetic construct
Residues:
QQYNSYPLT
9

Sequence Number (ID): 27
Length: 119
Molecule Type: AA
Features Location/Qualifiers:
- REGION, 1..119
> note, H0_15A7.5
- source, 1..119
> mol_type, protein
> organism, synthetic construct
Residues:
EVQLVESGGG LVQPGGSLRL SCAASGFTFS NYGMNWVRQA PGKGLEWVSF ISNLAYGINY
60
ADSVKGRFTI SRDNAKNSLY LQMNSLRAED TAVYYCARGA RATGWFAYWG QGTLVTVSS
119

Sequence Number (ID): 28
Length: 107
Molecule Type: AA
Features Location/Qualifiers:
- REGION, 1..107
> note, L0_15A7.5
- source, 1..107
> mol_type, protein
> organism, synthetic construct

Residues:
DIQMTQSPSS  LSASVGDRVT  ITCRASQNVD  THVAWFQQKP  GKAPKSLIYS  ASYLQSGVPS
60
RFSGSGSGTD  FTLTISSLQP  EDFATYYCQQ  YNSYPLTFGG  GTKVEIK
107

Sequence Number (ID): 29
Length: 8
Molecule Type: AA
Features Location/Qualifiers:
- REGION, 1..8
> note, CDR1 H1_15A7.5 IMGT
- source, 1..8
> mol_type, protein
> organism, synthetic construct
Residues:
GFTFSNYG
8

Sequence Number (ID): 30
Length: 8
Molecule Type: AA
Features Location/Qualifiers:
- REGION, 1..8
> note, CDR2 H1_15A7.5 IMGT
- source, 1..8
> mol_type, protein
> organism, synthetic construct
Residues:
ISNLAYGI
8

Sequence Number (ID): 31
Length: 12
Molecule Type: AA
Features Location/Qualifiers:
- REGION, 1..12
> note, CDR3 H1_15A7.5 IMGT
- source, 1..12
> mol_type, protein
> organism, synthetic construct
Residues:
ARGARATGWF  AY
12

Sequence Number (ID): 32
Length: 6
Molecule Type: AA
Features Location/Qualifiers:
- REGION, 1..6
> note, CDR1 L1_15A7.5 IMGT
- source, 1..6

> mol_type, protein
> organism, synthetic construct
Residues:
QNVDTH
6

Sequence Number (ID): 33
Residues:
000

Sequence Number (ID): 34
Length: 9
Molecule Type: AA
Features Location/Qualifiers:
- REGION, 1..9
> note, CDR3 L1_15A7.5 IMGT
- source, 1..9
> mol_type, protein
> organism, synthetic construct
Residues:
QQYNSYPLT
9

Sequence Number (ID): 35
Length: 5
Molecule Type: AA
Features Location/Qualifiers:
- REGION, 1..5
> note, CDR1 H1_15A7.5 Kabat
- source, 1..5
> mol_type, protein
> organism, synthetic construct
Residues:
NYGMA
5

Sequence Number (ID): 36
Length: 17
Molecule Type: AA
Features Location/Qualifiers:
- REGION, 1..17
> note, CDR2 H1_15A7.5 Kabat
- source, 1..17
> mol_type, protein
> organism, synthetic construct
Residues:
FISNLAYGIN  YADTVTG
17

Sequence Number (ID): 37
Length: 10

Molecule Type: AA
Features Location/Qualifiers:
- REGION, 1..10
> note, CDR3 H1_15A7.5 Kabat
- source, 1..10
> mol_type, protein
> organism, synthetic construct
Residues:
GARATGWFAY
10

Sequence Number (ID): 38
Length: 11
Molecule Type: AA
Features Location/Qualifiers:
- REGION, 1..11
> note, CDR1 L1_15A7.5 Kabat
- source, 1..11
> mol_type, protein
> organism, synthetic construct
Residues:
KASQNVDTHV A
11

Sequence Number (ID): 39
Length: 7
Molecule Type: AA
Features Location/Qualifiers:
- REGION, 1..7
> note, CDR2 L1_15A7.5 Kabat
- source, 1..7
> mol_type, protein
> organism, synthetic construct
Residues:
SASYRYS
7

Sequence Number (ID): 40
Length: 9
Molecule Type: AA
Features Location/Qualifiers:
- REGION, 1..9
> note, CDR3 L1_15A7.5 Kabat
- source, 1..9
> mol_type, protein
> organism, synthetic construct
Residues:
QQYNSYPLT
9

Sequence Number (ID): 41
Length: 119

Molecule Type: AA
Features Location/Qualifiers:
- REGION, 1..119
> note, H1_15A7.5
- source, 1..119
> mol_type, protein
> organism, synthetic construct
Residues:
EVQLVESGGG LVQPGGSLRL SCAASGFTFS NYGMAWVRQA PGKGLEWVSF ISNLAYGINY
60
ADTVTGRFTI SRDNAKNSLY LQMNSLRAED TAVYYCARGA RATGWFAYWG QGTLVTVSS
119

Sequence Number (ID): 42
Length: 108
Molecule Type: AA
Features Location/Qualifiers:
- REGION, 1..108
> note, L1_15A7.5
- source, 1..108
> mol_type, protein
> organism, synthetic construct
Residues:
DIQMTQSPSS LSASVGDRVT ITCCKASQNV DTHVAWFQQK PGKAPKSLIY SASYRYSGVP
60
SRFSGSGSGT DFTLTISSLQ PEDFATYYCQ QYNSYPLTFG GGTKVEIK
108

Sequence Number (ID): 43
Length: 8
Molecule Type: AA
Features Location/Qualifiers:
- REGION, 1..8
> note, CDR1 H2_15A7.5 IMGT
- source, 1..8
> mol_type, protein
> organism, synthetic construct
Residues:
GFTFSNYG
8

Sequence Number (ID): 44
Length: 8
Molecule Type: AA
Features Location/Qualifiers:
- REGION, 1..8
> note, CDR2 H2_15A7.5 IMGT
- source, 1..8
> mol_type, protein
> organism, synthetic construct
Residues:
ISNLAYGI

8

Sequence Number (ID): 45
Length: 12
Molecule Type: AA
Features Location/Qualifiers:
- REGION, 1..12
> note, CDR3 H2_15A7.5 IMGT
- source, 1..12
> mol_type, protein
> organism, synthetic construct
Residues:
ARGARATGWF  AY
12

Sequence Number (ID): 46
Length: 6
Molecule Type: AA
Features Location/Qualifiers:
- REGION, 1..6
> note, CDR1 L2_15A7.5 IMGT
- source, 1..6
> mol_type, protein
> organism, synthetic construct
Residues:
QNVDTH
6

Sequence Number (ID): 47
Residues:
000

Sequence Number (ID): 48
Length: 9
Molecule Type: AA
Features Location/Qualifiers:
- REGION, 1..9
> note, CDR3 L2_15A7.5 IMGT
- source, 1..9
> mol_type, protein
> organism, synthetic construct
Residues:
QQYNSYPLT
9

Sequence Number (ID): 49
Length: 5
Molecule Type: AA
Features Location/Qualifiers:
- REGION, 1..5
> note, CDR1 H2_15A7.5 Kabat

- source, 1..5
> mol_type, protein
> organism, synthetic construct
Residues:
NYGMA
5

Sequence Number (ID): 50
Length: 17
Molecule Type: AA
Features Location/Qualifiers:
- REGION, 1..17
> note, CDR2 H2_15A7.5 Kabat
- source, 1..17
> mol_type, protein
> organism, synthetic construct
Residues:
FISNLAYGIN  YADTVTG
17

Sequence Number (ID): 51
Length: 10
Molecule Type: AA
Features Location/Qualifiers:
- REGION, 1..10
> note, CDR3 H2_15A7.5 Kabat
- source, 1..10
> mol_type, protein
> organism, synthetic construct
Residues:
GARATGWFAY
10

Sequence Number (ID): 52
Length: 11
Molecule Type: AA
Features Location/Qualifiers:
- REGION, 1..11
> note, CDR1 L2_15A7.5 Kabat
- source, 1..11
> mol_type, protein
> organism, synthetic construct
Residues:
KASQNVDTHV  A
11

Sequence Number (ID): 53
Length: 7
Molecule Type: AA
Features Location/Qualifiers:
- REGION, 1..7
> note, CDR2 L2_15A7.5 Kabat

- source, 1..7
> mol_type, protein
> organism, synthetic construct
Residues:
SASYRYS
7

Sequence Number (ID): 54
Length: 9
Molecule Type: AA
Features Location/Qualifiers:
- REGION, 1..9
> note, CDR3 L2_15A7.5 Kabat
- source, 1..9
> mol_type, protein
> organism, synthetic construct
Residues:
QQYNSYPLT
9

Sequence Number (ID): 55
Length: 119
Molecule Type: AA
Features Location/Qualifiers:
- REGION, 1..119
> note, H2_15A7.5
- source, 1..119
> mol_type, protein
> organism, synthetic construct
Residues:
EVQLVESGGG LVQPGGSLRL SCAASGFTFS NYGMAWVRQA PGKGLEWVAF ISNLAYGINY
60
ADTVTGRFTI SRDNAKNSLY LQMNSLRAED TAVYYCARGA RATGWFAYWG QGTLVTVSS
119

Sequence Number (ID): 56
Length: 107
Molecule Type: AA
Features Location/Qualifiers:
- REGION, 1..107
> note, L2_15A7.5
- source, 1..107
> mol_type, protein
> organism, synthetic construct
Residues:
DIQMTQSPSS LSASVGDRVT ITCKASQNVD THVAWYQQKP GKAPKALIYS ASYRYSGVPS
60
RFSGSGSGTD FTLTISSLQP EDFATYYCQQ YNSYPLTFGG GTKVEIK
107

Sequence Number (ID): 57
Length: 8

Molecule Type: AA
Features Location/Qualifiers:
- REGION, 1..8
> note, CDR1 H3_15A7.5 IMGT
- source, 1..8
> mol_type, protein
> organism, synthetic construct
Residues:
GFTFSNYG
8

Sequence Number (ID): 58
Length: 8
Molecule Type: AA
Features Location/Qualifiers:
- REGION, 1..8
> note, CDR2 H3_15A7.5 IMGT
- source, 1..8
> mol_type, protein
> organism, synthetic construct
Residues:
ISNLAYGI
8

Sequence Number (ID): 59
Length: 12
Molecule Type: AA
Features Location/Qualifiers:
- REGION, 1..12
> note, CDR3 H3_15A7.5 IMGT
- source, 1..12
> mol_type, protein
> organism, synthetic construct
Residues:
ARGARATGWF  AY
12

Sequence Number (ID): 60
Length: 6
Molecule Type: AA
Features Location/Qualifiers:
- REGION, 1..6
> note, CDR1 L3_15A7.5 IMGT
- source, 1..6
> mol_type, protein
> organism, synthetic construct
Residues:
QNVDTH
6

Sequence Number (ID): 61
Residues:

000

Sequence Number (ID): 62
Length: 9
Molecule Type: AA
Features Location/Qualifiers:
- REGION, 1..9
> note, CDR3 L3_15A7.5 IMGT
- source, 1..9
> mol_type, protein
> organism, synthetic construct
Residues:
QQYNSYPLT
9

Sequence Number (ID): 63
Length: 5
Molecule Type: AA
Features Location/Qualifiers:
- REGION, 1..5
> note, CDR1 H3_15A7.5 Kabat
- source, 1..5
> mol_type, protein
> organism, synthetic construct
Residues:
NYGMA
5

Sequence Number (ID): 64
Length: 17
Molecule Type: AA
Features Location/Qualifiers:
- REGION, 1..17
> note, CDR2 H3_15A7.5 Kabat
- source, 1..17
> mol_type, protein
> organism, synthetic construct
Residues:
FISNLAYGIN  YADTVTG
17

Sequence Number (ID): 65
Length: 10
Molecule Type: AA
Features Location/Qualifiers:
- REGION, 1..10
> note, CDR3 H3_15A7.5 Kabat
- source, 1..10
> mol_type, protein
> organism, synthetic construct
Residues:

GARATGWFAY
10

Sequence Number (ID): 66
Length: 11
Molecule Type: AA
Features Location/Qualifiers:
- REGION, 1..11
> note, CDR1 L3_15A7.5 Kabat
- source, 1..11
> mol_type, protein
> organism, synthetic construct
Residues:
KASQNVDTHV A
11

Sequence Number (ID): 67
Length: 7
Molecule Type: AA
Features Location/Qualifiers:
- REGION, 1..7
> note, CDR2 L3_15A7.5 Kabat
- source, 1..7
> mol_type, protein
> organism, synthetic construct
Residues:
SASYRYS
7

Sequence Number (ID): 68
Length: 9
Molecule Type: AA
Features Location/Qualifiers:
- REGION, 1..9
> note, CDR3 L3_15A7.5 Kabat
- source, 1..9
> mol_type, protein
> organism, synthetic construct
Residues:
QQYNSYPLT
9

Sequence Number (ID): 69
Length: 119
Molecule Type: AA
Features Location/Qualifiers:
- REGION, 1..119
> note, H3_15A7.5
- source, 1..119
> mol_type, protein
> organism, synthetic construct
Residues:

EVQLVESGGG  LVQPGGSLRL  SCAASGFTFS  NYGMAWVRQA  PGKGPEWVAF  ISNLAYGINY
60
ADTVTGRFTI  SRDNAKNSLY  LQMNSLRAED  TAVYYCARGA  RATGWFAYWG  QGTLVTVSS
119

Sequence Number (ID): 70
Length: 108
Molecule Type: AA
Features Location/Qualifiers:
- REGION, 1..108
> note, L3_15A7.5
- source, 1..108
> mol_type, protein
> organism, synthetic construct
Residues:
DIQMTQSPSS  LSASVGDRVT  ITCKASQNVD  THVAWYYQQK  PGKSPKALIY  SASYRYSGVP
60
SRFSGSGSGT  DFTLTISSLQ  PEDFATYYCQ  QYNSYPLTFG  GGTKVEIK
108

Sequence Number (ID): 71
Length: 107
Molecule Type: AA
Features Location/Qualifiers:
- REGION, 1..107
> note, L3_15A7.5_mod
- source, 1..107
> mol_type, protein
> organism, synthetic construct
Residues:
DIQMTQSPSS  LSASVGDRVT  ITCKASQNVD  THVAWYYQQK  PGKSPKALIY  SASYRYSGVP
60
SRFSGSGSGT  DFTLTISSLQ  PEDFATYCQQ  YNSYPLTFGG  GTKVEIK
107

Sequence Number (ID): 72
Length: 8
Molecule Type: AA
Features Location/Qualifiers:
- REGION, 1..8
> note, CDR1 VH_HA22
- source, 1..8
> mol_type, protein
> organism, synthetic construct
Residues:
GFTFSSYN
8

Sequence Number (ID): 73
Length: 8
Molecule Type: AA
Features Location/Qualifiers:

- REGION, 1..8
        > note, CDR2 VH_HA22
- source, 1..8
        > mol_type, protein
        > organism, synthetic construct
Residues:
ISSSSSTI
8

Sequence Number (ID): 74
Length: 10
Molecule Type: AA
Features Location/Qualifiers:
- REGION, 1..10
        > note, CDR3 VH_HA22
- source, 1..10
        > mol_type, protein
        > organism, synthetic construct
Residues:
ARAYYYGMDV
10

Sequence Number (ID): 75
Length: 6
Molecule Type: AA
Features Location/Qualifiers:
- REGION, 1..6
        > note, CDR1 VK_HA22
- source, 1..6
        > mol_type, protein
        > organism, synthetic construct
Residues:
QGISGW
6

Sequence Number (ID): 76
Residues:
000

Sequence Number (ID): 77
Length: 9
Molecule Type: AA
Features Location/Qualifiers:
- REGION, 1..9
        > note, CDR3 VK_HA22
- source, 1..9
        > mol_type, protein
        > organism, synthetic construct
Residues:
QQANSFPPT
9

Sequence Number (ID): 78
Length: 117
Molecule Type: AA
Features Location/Qualifiers:
- REGION, 1..117
> note, VH_HA22
- source, 1..117
> mol_type, protein
> organism, synthetic construct
Residues:
EVQLVESGGG LVQPGGSLRL SCAASGFTFS SYNMNWVRQA PGKGLEWVSY ISSSSSTIYY
60
ADSVKGRFTI SRDNAKNSLS LQMNSLRDED TAVYYCARAY YYGMDVWGQG TTVTVSS
117

Sequence Number (ID): 79
Length: 107
Molecule Type: AA
Features Location/Qualifiers:
- REGION, 1..107
> note, VK_HA22
- source, 1..107
> mol_type, protein
> organism, synthetic construct
Residues:
DIQMTQSPSS VSASVGDRVT ITCRASQGIS GWLAWYQQKP GKAPKFLIYA ASTLQSGVPS
60
RFSGSGSGTD FTLTISSLQP EDFATYYCQQ ANSFPPTFGG GTKVEIK
107

Sequence Number (ID): 80
Length: 8
Molecule Type: AA
Features Location/Qualifiers:
- REGION, 1..8
> note, CDR1 VH_5A12.2
- source, 1..8
> mol_type, protein
> organism, synthetic construct
Residues:
GFTFNSMY
8

Sequence Number (ID): 81
Length: 8
Molecule Type: AA
Features Location/Qualifiers:
- REGION, 1..8
> note, CDR2 VH_5A12.2
- source, 1..8
> mol_type, protein

> organism, synthetic construct
Residues:
IYAGTGGT
8

Sequence Number (ID): 82
Length: 13
Molecule Type: AA
Features Location/Qualifiers:
- REGION, 1..13
> note, CDR3 VH_5A12.2
- source, 1..13
> mol_type, protein
> organism, synthetic construct
Residues:
AIRSGFVPMD YWG
13

Sequence Number (ID): 83
Length: 118
Molecule Type: AA
Features Location/Qualifiers:
- REGION, 1..118
> note, VH_5A12.2
- source, 1..118
> mol_type, protein
> organism, synthetic construct
Residues:
QIQLQQSGAE LVKPGASVTL SCKTSGFTFN SMYISWLKQK PGQSLEWIAW IYAGTGGTRF
60
NQKFTGKVQL TVDTSSSTAY MQFSSLTTDD SAIYYCAIRS GFVPMDYWGQ GTSVTVSS
118

Sequence Number (ID): 84
Length: 6
Molecule Type: AA
Features Location/Qualifiers:
- REGION, 1..6
> note, CDR1 VK_5A12.2
- source, 1..6
> mol_type, protein
> organism, synthetic construct
Residues:
QSVSND
6

Sequence Number (ID): 85
Residues:
000

Sequence Number (ID): 86

Length: 6
Molecule Type: AA
Features Location/Qualifiers:
- REGION, 1..6
> note, CDR3 VK_5A12.2
- source, 1..6
> mol_type, protein
> organism, synthetic construct
Residues:
QQDYSS
6

Sequence Number (ID): 87
Length: 107
Molecule Type: AA
Features Location/Qualifiers:
- REGION, 1..107
> note, VK_5A12.2
- source, 1..107
> mol_type, protein
> organism, synthetic construct
Residues:
SIVMTQTPKF LLVSAGDRVT ITCKASQSVS NDVAWYQQKP GQSPKLLIYY ASNRYTGVPD
60
RFTGSGYGTD FTFTISTVQA EDLAVYFCQQ DYSSPWTFGG GTKLEIK
107

Sequence Number (ID): 88
Length: 8
Molecule Type: AA
Features Location/Qualifiers:
- REGION, 1..8
> note, CDR1 VH_9A2.7
- source, 1..8
> mol_type, protein
> organism, synthetic construct
Residues:
GYNFTTFW
8

Sequence Number (ID): 89
Length: 8
Molecule Type: AA
Features Location/Qualifiers:
- REGION, 1..8
> note, CDR2 VH_9A2.7
- source, 1..8
> mol_type, protein
> organism, synthetic construct
Residues:
IYPSDSYA
8

Sequence Number (ID): 90
Length: 13
Molecule Type: AA
Features Location/Qualifiers:
- REGION, 1..13
> note, CDR3 VH_9A2.7
- source, 1..13
> mol_type, protein
> organism, synthetic construct
Residues:
TRPSYFGRNS FAY
13

Sequence Number (ID): 91
Length: 120
Molecule Type: AA
Features Location/Qualifiers:
- REGION, 1..120
> note, VH_9A2.7
- source, 1..120
> mol_type, protein
> organism, synthetic construct
Residues:
QVQLQQPGAE LVRPGASVKL SCKASGYNFT TFWINWVKQR PGQGLEWIGN IYPSDSYANY
60
NQKFKDKATL TVDKSSTTAY MQLSSPTSED SAVYYCTRPS YFGRNSFAYW GQGTLVTVSA
120

Sequence Number (ID): 92
Length: 12
Molecule Type: AA
Features Location/Qualifiers:
- REGION, 1..12
> note, CDR1 VK_9A2.7
- source, 1..12
> mol_type, protein
> organism, synthetic construct
Residues:
QSLLYSVNHK NY
12

Sequence Number (ID): 93
Residues:
000

Sequence Number (ID): 94
Length: 9
Molecule Type: AA
Features Location/Qualifiers:
- REGION, 1..9

                    > note, CDR3 VK_9A2.7
          - source, 1..9
                    > mol_type, protein
                    > organism, synthetic construct
     Residues:
     HQYYTYPLT
9

     Sequence Number (ID): 95
     Length: 113
     Molecule Type: AA
     Features Location/Qualifiers:
          - REGION, 1..113
                    > note, VK_9A2.7
          - source, 1..113
                    > mol_type, protein
                    > organism, synthetic construct
     Residues:
     DIVMTQSPSS  LVVSVGEKVT  MSCKSSQSLL  YSVNHKNYLA  WYQQKPGQSP  KLLIYWASTR
60
     ESGVPDRFTG  SGSGTDFSLT  ISSVKAEDLA  VYYCHQYYTY  PLTFGAGTKL  ELK
113

     Sequence Number (ID): 96
     Length: 8
     Molecule Type: AA
     Features Location/Qualifiers:
          - REGION, 1..8
                    > note, CDR1 VH_3A1.4
          - source, 1..8
                    > mol_type, protein
                    > organism, synthetic construct
     Residues:
     GDSITSGY
8

     Sequence Number (ID): 97
     Length: 7
     Molecule Type: AA
     Features Location/Qualifiers:
          - REGION, 1..7
                    > note, CDR2 VH_3A1.4
          - source, 1..7
                    > mol_type, protein
                    > organism, synthetic construct
     Residues:
     ISNSGIT
7

     Sequence Number (ID): 98
     Length: 15
     Molecule Type: AA

Features Location/Qualifiers:
        - REGION, 1..15
                > note, CDR3 VH_3A1.4
        - source, 1..15
                > mol_type, protein
                > organism, synthetic construct
Residues:
TRFGSTMILY  YTMDY
15

Sequence Number (ID): 99
Length: 121
Molecule Type: AA
Features Location/Qualifiers:
        - REGION, 1..121
                > note, VH_3A1.4
        - source, 1..121
                > mol_type, protein
                > organism, synthetic construct
Residues:
DVQLQESGPS  LVKPSQTLSL  TCSVTGDSIT  SGYWNWIRKF  PGNKLEYMGY  ISNSGITYYN
60
PSLKSRISIT  RDTSKNQYFL  QLNSVTAEDT  ATYYCTRFGS  TMILYYTMDY  WGQGTSVTVS
120
S
121

Sequence Number (ID): 100
Length: 6
Molecule Type: AA
Features Location/Qualifiers:
        - REGION, 1..6
                > note, CDR1 VK_3A1.4
        - source, 1..6
                > mol_type, protein
                > organism, synthetic construct
Residues:
QSISDY
6

Sequence Number (ID): 101
Residues:
000

Sequence Number (ID): 102
Length: 9
Molecule Type: AA
Features Location/Qualifiers:
        - REGION, 1..9
                > note, CDR3 VK_3A1.4
        - source, 1..9

>                    > mol_type, protein
                    > organism, synthetic construct
        Residues:
        QNGHSFPLT
9

        Sequence Number (ID): 103
        Length: 107
        Molecule Type: AA
        Features Location/Qualifiers:
                - REGION, 1..107
                        > note, VK_3A1.4
                - source, 1..107
                        > mol_type, protein
                        > organism, synthetic construct
        Residues:
        DIVMTQSPVT  LSVTPGDRVS  LSCRASQSIS  DYLHWYQQKS  QESPRLLIKY  ASKSISGIPS
60
        RFSGSGSGSN  FTLSINSVEP  EDVGVYYCQN  GHSFPLTFGA  GTKLELK
107

        Sequence Number (ID): 104
        Length: 8
        Molecule Type: AA
        Features Location/Qualifiers:
                - REGION, 1..8
                        > note, CDR1 VH_8F06
                - source, 1..8
                        > mol_type, protein
                        > organism, synthetic construct
        Residues:
        GYTFTSYV
8

        Sequence Number (ID): 105
        Length: 8
        Molecule Type: AA
        Features Location/Qualifiers:
                - REGION, 1..8
                        > note, CDR2 VH_8F06
                - source, 1..8
                        > mol_type, protein
                        > organism, synthetic construct
        Residues:
        INPYNDGT
8

        Sequence Number (ID): 106
        Length: 6
        Molecule Type: AA
        Features Location/Qualifiers:
                - REGION, 1..6

> note, CDR3 VH_8F06
- source, 1..6
> mol_type, protein
> organism, synthetic construct
Residues:
VTLFAY
6

Sequence Number (ID): 107
Length: 113
Molecule Type: AA
Features Location/Qualifiers:
- REGION, 1..113
> note, VH_8F06
- source, 1..113
> mol_type, protein
> organism, synthetic construct
Residues:
EVQLQQSGPE LVKPGASVKM SCKASGYTFT SYVMHWVKQK PGQGLEWIGY INPYNDGTKY
60
NEKFKGKATL TSDKSSSTAY MELSSLTSED SAVYYCVTLF AYWGQGTLVT VSA
113

Sequence Number (ID): 108
Length: 5
Molecule Type: AA
Features Location/Qualifiers:
- REGION, 1..5
> note, CDR1 VK_8F06
- source, 1..5
> mol_type, protein
> organism, synthetic construct
Residues:
SSISY
5

Sequence Number (ID): 109
Residues:
000

Sequence Number (ID): 110
Length: 9
Molecule Type: AA
Features Location/Qualifiers:
- REGION, 1..9
> note, CDR3 VK_8F06
- source, 1..9
> mol_type, protein
> organism, synthetic construct
Residues:
HQRSSYPFT

9

Sequence Number (ID): 111
Length: 106
Molecule Type: AA
Features Location/Qualifiers:
- REGION, 1..106
> note, VK_8F06
- source, 1..106
> mol_type, protein
> organism, synthetic construct
Residues:
QIVLTQSPAI MSASPGEKVT MTCSASSSIS YMHWYQQKPG TSPKRWIYDT SKLASGVPAR
60
FSGSGSGTSY SLTISSMEAE DAATYYCHQR SSYPFTFGSG TKLEIK
106

Sequence Number (ID): 112
Length: 4
Molecule Type: AA
Features Location/Qualifiers:
- source, 1..4
> mol_type, protein
> organism, synthetic construct
Residues:
GAGG
4

Sequence Number (ID): 113
Length: 4
Molecule Type: AA
Features Location/Qualifiers:
- source, 1..4
> mol_type, protein
> organism, synthetic construct
Residues:
GGAG
4

Sequence Number (ID): 114
Length: 4
Molecule Type: AA
Features Location/Qualifiers:
- source, 1..4
> mol_type, protein
> organism, synthetic construct
Residues:
GVGG
4

Sequence Number (ID): 115
Length: 4

Molecule Type: AA
Features Location/Qualifiers:
- source, 1..4
> mol_type, protein
> organism, synthetic construct
Residues:
GGVG
4

Sequence Number (ID): 116
Length: 4
Molecule Type: AA
Features Location/Qualifiers:
- source, 1..4
> mol_type, protein
> organism, synthetic construct
Residues:
GGFG
4

Sequence Number (ID): 117
Length: 4
Molecule Type: AA
Features Location/Qualifiers:
- source, 1..4
> mol_type, protein
> organism, synthetic construct
Residues:
GFGG
4

Sequence Number (ID): 118
Length: 4
Molecule Type: AA
Features Location/Qualifiers:
- source, 1..4
> mol_type, protein
> organism, synthetic construct
Residues:
GGSG
4

Sequence Number (ID): 119
Length: 5
Molecule Type: AA
Features Location/Qualifiers:
- source, 1..5
> mol_type, protein
> organism, synthetic construct
Residues:
GGSGG
5

Sequence Number (ID): 120
Length: 5
Molecule Type: AA
Features Location/Qualifiers:
- source, 1..5
> mol_type, protein
> organism, synthetic construct
Residues:
GSGSG
5

Sequence Number (ID): 121
Length: 5
Molecule Type: AA
Features Location/Qualifiers:
- source, 1..5
> mol_type, protein
> organism, synthetic construct
Residues:
GSGGG
5

Sequence Number (ID): 122
Length: 5
Molecule Type: AA
Features Location/Qualifiers:
- source, 1..5
> mol_type, protein
> organism, synthetic construct
Residues:
GGGSG
5

Sequence Number (ID): 123
Length: 5
Molecule Type: AA
Features Location/Qualifiers:
- source, 1..5
> mol_type, protein
> organism, synthetic construct
Residues:
GSSSG
5
END

## Claims

1. An antibody-drug conjugate of Formula I, or a pharmaceutically acceptable salt thereof,
D-M-V (I),
wherein
D is Belotecan or a derivative thereof represented by formula (II) 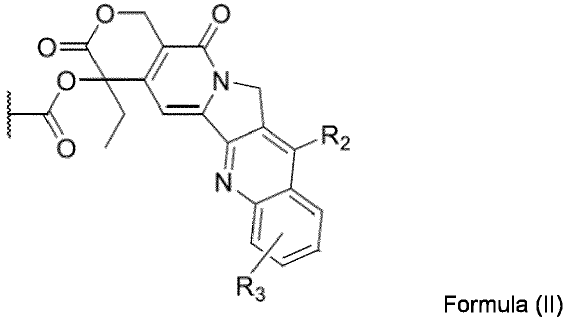
wherein
R² is -(CH₂)₂NR¹CH(CH₃)₂,
R¹ is H or -SO₂CH₃, and
R³ is H, amino, or C₁-C₆ alkyl, preferably H
M is a linker conjugating D to V, and
V is an anti-Nectin-4 antibody or antigen-binding fragment thereof.

2. The antibody-drug conjugate of claim 1,
wherein M is a cleavable linker, and/or
wherein M is a hydrophilic polysarcosine linker, a hydrophilic linker comprising at least one ethylene glycol unit, a linker comprising a highly polar spacer, or an oligopeptide linker.

3. The antibody-drug conjugate of claim 1,
wherein M is a hydrophilic polysarcosine linker comprising, e.g., about 8-12 sarcosine units, and at least one ethylene glycol unit, e.g., 1, 2, 3, 4, 5, 6, 7, 8, 9, or 10 or more ethylene glycol units, or
wherein M is a linker comprising Val-Ala or GGFG and optionally at least one ethylene glycol unit, e.g., 1, 2, 3, 4, 5, 6, 7, 8, 9, or 10 or more ethylene glycol units, in particular a Val-Ala or GGFG linker with 8 ethylene glycol units.

4. The antibody-drug conjugate of any one of claims 1 to 3,
wherein the linker is a hydrophilic polysarcosine linker comprising 10 sarcosine units and 2 ethylene glycol units.

5. The antibody-drug conjugate of any one of claims 1-4,
wherein the linker is subject to cleavage by a glycosidase, and particularly subject to cleavage by a glucuronidase.

6. The antibody-drug conjugate of any one of claims 1-5,
wherein the anti-Nectin-4 antibody or antigen-binding fragment thereof is selected from a chimeric antibody, a multispecific antibody, in particular a bispecific antibody, a human antibody, a humanized antibody, or an antigen-binding fragment thereof.

7. The antibody-drug conjugate of any one of claims 1 to 6,
wherein the anti-Nectin-4 antibody or antigen-binding fragment thereof is an antibody selected from an antibody of class IgG, e.g., of subclass IgG1, IgG2, IgG3 of IgG4, of class IgM, of class IgA or an antigen-binding fragment thereof, or a single-chain antibody, or an antibody Fv fragment, wherein the antibody optionally has a heavy chain constant domain having a reduced effector function, e.g., which has a reduced binding to the Fc receptor.

8. The antibody-drug conjugate of any one of claims 1 to 7,
wherein the anti-Nectin-4 antibody or antigen-binding fragment thereof specifically binds to Nectin-4 expressed by tumors with a higher affinity in comparison to Nectin-4 expressed by human differentiated keratinocytes, or an antigen-binding fragment thereof.

9. The antibody-drug conjugate of any one of claims 1-8 comprising an anti-Nectin-4 antibody or antigen-binding fragment thereof comprising:
(a) a variable heavy chain (VH) region comprising complementarity-determining regions (CDRs) CDR-H1, CDR-H2 and CDR-H3, wherein
(i) the CDR-H1 comprises an amino acid sequence according to SEQ ID NO: 7, 72, 80, 88, 96 or 104,
or an amino acid sequence comprising a substitution or insertion, particularly a conservative substitution and/or histidine insertion of at least one amino acid, e.g., 1 or 2 amino acids,
(ii) the CDR-H2 comprises an amino acid sequence according to SEQ ID NO: 8, 73, 81, 89, 97 or 105,
or an amino acid sequence comprising a substitution or insertion, particularly a conservative substitution and/or histidine insertion of at least one amino acid, e.g., 1 or 2 amino acids,
(iii) the CDR-H3 comprises an amino acid sequence according to SEQ ID NO: 9, 74, 82, 90, 98 or 106,
or an amino acid sequence comprising a substitution or insertion, particularly a conservative substitution and/or histidine insertion of at least one amino acid, e.g., 1 or 2 amino acids,
and optionally
(b) a variable light chain (VL) region, particularly a VL region comprising complementarity-determining regions (CDRs) CDR-L1, CDR-L2 and CDR-L3, wherein
(i) the CDR-L1 comprises an amino acid sequence according to SEQ ID NO: 10, 75, 84, 92, 100 or 108,
or an amino acid sequence comprising a substitution or insertion, particularly a conservative substitution and/or histidine insertion of at least one amino acid, e.g., 1 or 2 amino acids,
(ii) the CDR-L2 comprises an amino acid sequence according to SEQ ID NO: 11, 76, 85, 93, 101 or 109,
or an amino acid sequence comprising a substitution or insertion, particularly a conservative substitution and/or histidine insertion of at least one amino acid, e.g., 1 or 2 amino acids,
(iii) the CDR-L3 comprises an amino acid sequence according to SEQ ID NO: 12, 77, 86, 94, 102 or 110,
or an amino acid sequence comprising a substitution or insertion, particularly a conservative substitution and/or histidine insertion of at least one amino acid, e.g., 1 or 2 amino acids.

10. The antibody-drug conjugate of any one of claims 1-9 comprising an antibody or antigen-binding fragment thereof comprising:
(a)
(i) a VH region comprising the CDR-H1 of SEQ ID NO:7, the CDR-H2 of SEQ ID NO:8, and the CDR-H3 of SEQ ID NO:9, and optionally
(ii) a VL region comprising the CDR-L1 of SEQ ID NO:10, the CDR-L2 of the SEQ ID NO:11, and the CDR-L3 of the SEQ ID NO:12,
or
(b)
(i) a VH region comprising the CDR-H1 of SEQ ID NO:88, the CDR-H2 of SEQ ID NO:89, and the CDR-H3 of SEQ ID NO:90, and optionally
(ii) a VL region comprising the CDR-L1 of SEQ ID NO:92, the CDR-L2 of the SEQ ID NO:93, and the CDR-L3 of the SEQ ID NO:94,
or
(c)
(i) a VH region comprising the CDR-H1 of SEQ ID NO:96, the CDR-H2 of SEQ ID NO:97, and the CDR-H3 of SEQ ID NO:98, and optionally
(ii) a VL region comprising the CDR-L1 of SEQ ID NO:100, the CDR-L2 of the SEQ ID NO:101, and the CDR-L3 of the SEQ ID NO:102,
or
(d)
(i) a VH region comprising the CDR-H1 of SEQ ID NO:104, the CDR-H2 of SEQ ID NO:105, and the CDR-H3 of SEQ ID NO:106, and optionally
(ii) a VL region comprising the CDR-L1 of SEQ ID NO:108, the CDR-L2 of the SEQ ID NO:109, and the CDR-L3 of the SEQ ID NO:110,
or
(e)
(i) a VH region comprising the CDR-H1 of SEQ ID NO:72, the CDR-H2 of SEQ ID NO:73, and the CDR-H3 of SEQ ID NO:74, and optionally
(ii) a VL region comprising the CDR-L1 of SEQ ID NO:75, the CDR-L2 of the SEQ ID NO:76, and the CDR-L3 of the SEQ ID NO:77,
or
(f)
(i) a VH region comprising the CDR-H1 of SEQ ID NO:80, the CDR-H2 of SEQ ID NO:81, and the CDR-H3 of SEQ ID NO:82, and optionally
(ii) a VL region comprising the CDR-L1 of SEQ ID NO:84, the CDR-L2 of the SEQ ID NO:85, and the CDR-L3 of the SEQ ID NO:86.

11. The antibody-drug conjugate of any one of claims 1-10 comprising an antibody or antigen-binding fragment thereof comprising:
(a) a VH region comprising an amino acid sequence according to SEQ ID NO:13, 78, 83, 91, 99 or 107 or an amino acid sequence having an identity thereof of at least 85%, at least 90%, at least 95% or at least 99%,
and optionally
(b) a VL region, particularly a VL region comprising an amino acid sequence according to SEQ ID NO:14, 79, 87, 95, 103 or 111 or an amino sequence having an identity thereof of at least 85%, at least 90%, at least 95% or at least 99%.

12. The antibody-drug conjugate of any one of claims 1-11 comprising an antibody or antigen-binding fragment thereof comprising:
(a)
(i) a VH region comprising an amino acid sequence according to SEQ ID NO:13 or an amino acid sequence having an identity thereof of at least 85%, at least 90%, at least 95% or at least 99%,
and optionally
(ii) a VL region, particularly a VL region comprising an amino acid sequence according to SEQ ID NO:14 or an amino sequence having an identity thereof of at least 85%, at least 90%, at least 95% or at least 99%,
or
(b)
(i) a VH region comprising an amino acid sequence according to SEQ ID NO:91 or an amino acid sequence having an identity thereof of at least 85%, at least 90%, at least 95% or at least 99%,
and optionally
(ii) a VL region, particularly a VL region comprising an amino acid sequence according to SEQ ID NO:95 or an amino sequence having an identity thereof of at least 85%, at least 90%, at least 95% or at least 99%,
or
(c)
(i) a VH region comprising an amino acid sequence according to SEQ ID NO:99 or an amino acid sequence having an identity thereof of at least 85%, at least 90%, at least 95% or at least 99%,
and optionally
(ii) a VL region, particularly a VL region comprising an amino acid sequence according to SEQ ID NO:103 or an amino sequence having an identity thereof of at least 85%, at least 90%, at least 95% or at least 99%,
or
(d)
(i) a VH region comprising an amino acid sequence according to SEQ ID NO:107 or an amino acid sequence having an identity thereof of at least 85%, at least 90%, at least 95% or at least 99%,
and optionally
(ii) a VL region, particularly a VL region comprising an amino acid sequence according to SEQ ID NO:111 or an amino sequence having an identity thereof of at least 85%, at least 90%, at least 95% or at least 99%,
or
(e)
(i) a VH region comprising an amino acid sequence according to SEQ ID NO:78 or an amino acid sequence having an identity thereof of at least 85%, at least 90%, at least 95% or at least 99%,
and optionally
(ii) a VL region, particularly a VL region comprising an amino acid sequence according to SEQ ID NO:79 or an amino sequence having an identity thereof of at least 85%, at least 90%, at least 95% or at least 99%,
or
(f)
(i) a VH region comprising an amino acid sequence according to SEQ ID NO:83 or an amino acid sequence having an identity thereof of at least 85%, at least 90%, at least 95% or at least 99%,
and optionally
(ii) a VL region, particularly a VL region comprising an amino acid sequence according to SEQ ID NO:87 or an amino sequence having an identity thereof of at least 85%, at least 90%, at least 95% or at least 99%.

13. A pharmaceutical composition comprising an active agent, which is an antibody-drug conjugate according to any one of claims 1-12, and a pharmaceutically acceptable carrier and/or excipient.

14. An antibody-drug conjugate of any one of claims 1-12 or a pharmaceutical composition of claim 13 for use in medicine, particularly in human medicine.

15. An antibody-drug conjugate of any one of claims 1-12 or a pharmaceutical composition of claim 13 for use in the prevention and/or treatment of a Nectin-4 associated disorder, particularly for the prevention and/or the treatment of a Nectin-4 positive cancer, particularly for the prevention and/or the treatment of a cancer selected from bladder, urothelial, endometrial, cervical, colorectal, liver, thyroid, breast, pancreatic, lung, ovarian, head and neck and/or esophagus cancer.
